# EUROPEAN PATENT APPLICATION

(11) **EP 4 725 301 A1**
(43) Date of publication of application: **15.04.2026**
(21) Application number: 25201839.5
(22) Date of filing: 12.09.2025
(51) Int. Cl.: A01H 1/00, A01H 5/10, A01H 6/66, A01H 1/04

(54) **SESAME AND SESAME PRODUCTS WITH IMPROVED TASTE AND ENHANCED NUTRITIONAL VALUE, FROM SHATTER RESISTANT SESAME PLANTS**

(30) Priority: 12.09.2024 US 202463693733 P; 16.05.2025 US 202563807086 P; 04.07.2025 JP 2025113490
(71) Applicant: Equi-Nom Ltd., Givat Brenner 6094800 (IL)
(72) Inventor: ZEMACH, Itay, 7628708 Rehovot (IL); SKLARZ, Menachem, 8473524 Beer Sheva (IL); GAR, Oron, 7935100 Lachish Darom (IL); BARDANOV, Nimrod, 8547000 Sde Nitzan 57 (IL)
(74) Representative: Pearl Cohen UK

(57) **Abstract**

Sesame plants with high protein content and/or improved taste, as well as seeds thereof and food products made therefrom are provided. Phenotypic and genotypic analysis of many sesame varieties were performed followed by analysis to derive markers for phenotypic traits that contribute to high protein content and/or improved taste, and a breeding simulation was used to identify the most common and most stable markers. Following verification of trait stability over several generations, markers and marker cassettes were defined as being uniquely present in the developed sesame lines. Various food products made of seeds of the disclosed varieties have higher protein content and improved taste compared to products made of commercial sesame varieties.

## Description

### SEQUENCE LISTING STATEMENT

The instant application contains a Sequence Listing which has been submitted electronically in XML format and is hereby incorporated by reference in its entirety. The XML format copy, created on September 9, 2025, is named P-612941-EP_ST26.xml, and is 44 KB in size.

### BACKGROUND OF THE INVENTION

### 1. TECHNICAL FIELD

The present invention relates to the field of sesame breeding and more particularly, to quantitative trait loci (QTLs, or QTL) associated with sesame protein composition and sesame taste.

### 2. DISCUSSION OF RELATED ART

Sesame (*Sesamum indicum*) is an oilseed crop that is cultivated over a large range of soil and climate conditions, typically in subtropical climates, and is used for seeds, oil and paste products.

U.S. Patent No. 10,577,623, which is incorporated herein by reference in its entirety, discloses genetic improvement for shatter resistant capsules, and more specifically, novel QTL conferring shatter resistant capsules, and methods for introgressing and combining novel QTL into elite germplasm in a breeding program for shatter resistant capsules.

Boydak et al. 2008 ("Effect of varieties and years on seed composition of sesame (Sesamum indicum L.) grown in semi-arid area", Asian Journal of Chemistry 20(5): 3907-3912), which is incorporated herein by reference in its entirety, discloses the effect of variety and growing year on oil, protein and fatty acid composition of five sesame cultivars. Of the five sesame cultivars, the sesame variety Muganli-57 studied in Boydak *et al.* 2008, which does not include the genetic markers disclosed below (and the respective results) - was verified to have the performance described in Boydak *et al.* 2008 and is used herein as a baseline reference. The inventors note that the Muganli-57 variety already has an improved taste compared with a common commercial variety, the Ethiopian line "Humera", which scored almost two grades in the tasting tests below the Muganli-57 variety.

### SUMMARY OF THE INVENTION

The following is a simplified summary providing an initial understanding of the invention. The summary does not necessarily identify key elements nor limit the scope of the invention, but merely serves as an introduction to the following description.

One aspect of the present invention provides sesame plants with shatter-resistant capsules, progeny thereof, seeds and/or part(s) thereof, the sesame plant comprising a plurality of quantitative trait loci (QTLs) having a corresponding plurality of nucleic acid genetic markers that are associated with a plurality of phenotypic traits concerning taste and nutritional content of the seeds, wherein the QTLs are combined in the sesame plant from a plurality of sesame varieties by computationally supported breeding, wherein the QTLs comprise QTL 1 associated with improved taste, with corresponding markers set forth in SEQ ID NOs 1 or 2, wherein the sesame plant or part thereof is homozygous or heterozygous with respect to SEQ ID NO 1 and at least one additional QTL, and wherein the markers are arranged in cassettes comprising at least one of: cassettes 1, 2, 3 or 4, comprising at least QTL 1, and QTL 2 associated with lowered ash content and increased protein level, with corresponding markers set forth in SEQ ID NOs 3 or 4, and wherein the sesame plant or part thereof is homozygous or heterozygous with respect to SEQ ID NO 3; cassette 5 comprising at least QTL 1, and QTLs 8 and 9 associated with high protein content, with QTL 8 having corresponding markers set forth in SEQ ID NOs 15 or 16, wherein the sesame plant or part thereof is homozygous or heterozygous with respect to SEQ ID NO 15, and with QTL 9 having corresponding markers set forth in SEQ ID NOs 17 or 18, wherein the sesame plant or part thereof is homozygous or heterozygous with respect to SEQ ID NO 17; cassette 6 comprising at least QTL 1, and QTL 10 associated with improved taste, with corresponding markers set forth in SEQ ID NOs 19 or 20, wherein the sesame plant or part thereof is homozygous or heterozygous with respect to SEQ ID NO 19; and/or cassette 7 comprising at least QTL 1, and QTL 11 and QTL 12 associated with high protein content, with QTL 11 having corresponding markers set forth in SEQ ID NOs 21 or 22, wherein the sesame plant or part thereof is homozygous or heterozygous with respect to SEQ ID NO 21 and with QTL 12 having corresponding markers set forth in SEQ ID NOs 23 or 24, wherein the sesame plant or part thereof is homozygous or heterozygous with respect to SEQ ID NO 23.

One aspect of the present invention provides sesame plants with shatter-resistant capsules, progeny thereof, seeds and/or part(s) thereof, the sesame plant comprising: a plurality of quantitative trait loci (QTLs) having a corresponding plurality of nucleic acid genetic markers that are associated with a plurality of phenotypic traits concerning taste and nutritional content of the seeds, wherein the QTLs are combined in the sesame plant from a plurality of sesame varieties by computationally supported breeding, wherein the QTLs comprise QTL 2 associated with lowered ash content with corresponding markers set forth in SEQ ID NOs 3 or 4, wherein the sesame plant or part thereof is homozygous or heterozygous with respect to SEQ ID NO 3, and at least one additional QTL, and wherein the markers are arranged in cassettes comprising at least one of cassettes 1, 2, 3 or 4, comprising at least QTL 2, and QTL 1 associated with improved taste, with corresponding markers set forth in SEQ ID NOs 1 or 2, wherein the sesame plant or part thereof is homozygous or heterozygous with respect to SEQ ID NO 1.

One aspect of the present invention provides food products, prepared with seeds of the disclosed sesame plants and comprising at least one of the markers, the food product comprising at least one of: non-viable sesame seeds, dehulled seeds, baked or fried goods including and/or topped with the sesame seeds or dehulled seeds, food and protein bars including the seeds, granola mixes and food bars including the seeds, tahini and spreads, dips and sauces made of or including the seeds, alternative milk products made of or including the seeds, and halva, candy and confectionary made of or including the seeds.

These, additional, and/or other aspects and/or advantages of the present invention are set forth in the detailed description which follows; possibly inferable from the detailed description; and/or learnable by practice of the present invention.

### BRIEF DESCRIPTION OF THE DRAWINGS

For a better understanding of embodiments of the invention and to show how the same may be carried into effect, reference will now be made, purely by way of example, to the accompanying drawings in which like numerals designate corresponding elements or sections throughout. In the accompanying drawings:
**Figure 1** is a high-level schematic illustration of sesame chromosomes with indications of the relevant markers' loci, according to some embodiments of the invention.
**Figure 2A** is a high-level schematic illustration of a computationally supported breeding method, according to some embodiments of the invention.
**Figure 2B** provides a non-limiting example of the breeding process and genealogy of one of the disclosed varieties, developed using the disclosed methodology, according to some embodiments of the invention.
**Figure 3A** illustrates the effects of the marker states of QTL 2 on the protein level in disclosed varieties, according to some embodiments of the invention.
**Figure 3B** illustrates the effects of the marker states of QTL 3 on the methionine level in disclosed varieties, according to some embodiments of the invention.
**Figures 4A-4D** illustrate the relation between multiple parameters and bitterness, according to some embodiments of the invention.
**Figures 4E** and **4F** illustrate the corresponding effect of QTL 1 on the taste of seeds and tahini prepared from seeds of the disclosed varieties, respectively, according to some embodiments of the invention.
**Figures 5A-5H** illustrate the improved taste of seeds from disclosed varieties with each of the marker cassettes, according to some embodiments of the invention.
**Figure 6** illustrates the relative protein content of disclosed sesame lines compared to the Muganli-57 sesame variety, according to some embodiments of the invention.
**Figure 7** illustrates the retention and detection of genomic DNA markers in tahini following industrial processing, according to some embodiments of the invention.

It will be appreciated that for simplicity and clarity of illustration, elements shown in the figures have not necessarily been drawn to scale. For example, the dimensions of some of the elements may be exaggerated relative to other elements for clarity. Further, where considered appropriate, reference numerals may be repeated among the figures to indicate corresponding or analogous elements.

### DETAILED DESCRIPTION OF THE INVENTION

In the following description, various aspects of the present invention are described. For purposes of explanation, specific configurations and details are set forth in order to provide a thorough understanding of the present invention. However, it will also be apparent to one skilled in the art that the present invention may be practiced without the specific details presented herein. Furthermore, well known features may have been omitted or simplified in order not to obscure the present invention. With specific reference to the drawings, it is stressed that the particulars shown are by way of example and for purposes of illustrative discussion of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

Before at least one embodiment of the invention is explained in detail, it is to be understood that the invention is not limited in its application to the details of construction and the arrangement of the components set forth in the following description or illustrated in the drawings. The invention is applicable to other embodiments that may be practiced or carried out in various ways as well as to combinations of the disclosed embodiments. Also, it is to be understood that the phraseology and terminology employed herein are for the purpose of description and should not be regarded as limiting.

Unless specifically stated otherwise, as apparent from the following discussions, it is appreciated that throughout the specification discussions utilizing terms such as "processing", "computing", "calculating", "determining", "enhancing", "deriving" or the like, refer to the action and/or processes of a computer or computing system, or similar electronic computing device, that manipulates and/or transforms data represented as physical, such as electronic, quantities within the computing system's registers and/or memories into other data similarly represented as physical quantities within the computing system's memories, registers or other such information storage, transmission or display devices.

Sesame plants with high protein content and/or improved taste, as well as seeds thereof and food products made therefrom are provided. Phenotypic and genotypic analysis of many sesame varieties were performed followed by analysis to derive markers for phenotypic traits that contribute to high protein content and/or improved taste, and a breeding simulation was used to identify the most common and most stable markers. Following verification of trait stability over several generations, markers and marker cassettes were defined as being uniquely present in the developed sesame lines. Various food products made of seeds of the disclosed varieties have higher protein content and improved taste compared to products made of commercial sesame varieties.

Embodiments of the present invention include various food products prepared from sesame of the disclosed sesame varieties. The advantages in taste achieved through the breeding of the varieties were found to be present also in food products prepared from the sesame seeds. In most of the food products, the inventors have found out that the disclosed markers are still present and can be detected in the food products, as indicated by the experimental results included herein. Non-limiting examples for food products include sesame and dehulled (non-viable and non-germinating) sesame seeds, packaged and sold as seeds, used as topping and/or added to a variety of baked goods or fried goods (e.g., buns, bagels, snacks, etc.), food and protein bars, granola mixtures and granola bars, various types of candy, various types of spreads (e.g., tahini, hummus, etc.), confectionary foods (e.g., halva), various food products prepared from ground or broken sesame, sesame flour and/or sesame oil, as well as sesame foul and sesame oil themselves. Following initial investigation, all processed sesame retains detectable genetic markers disclosed herein, probably except for products which are heat processed (e.g., sesame oil), as heat is expected to degrade genetic material.

Advantageously, sesame flour and sesame protein concentrate or isolate contain high amounts of sulfur-containing amino acids (e.g., methionine and cysteine), while most legume and cereal crops, such as pea, soybean, wheat, barley, rice, corn and others are low in sulfur-containing amino acids. Therefore, sesame can be used as a complementary supplement to cereal and legume proteins. For example, disclosed sesame variety products may be mixed with products from legume and/or cereal crops to yield products which have improved nutritional value and improved quantitative relations among nutritional elements. Moreover, mixing sesame products with cereal/legume products may improve the taste and other culinary traits.

U.S. Patent No. 10,577,623 and U.S. Patent Application Publication No. 2020/0093087, which are incorporated herein by reference in their entirety, teach QTLs that confer shatter resistant capsules, and elite sesame varieties having shatter resistant capsules. Shatter resistant lines were used at least partly to further derive the disclosed high protein sesame lines disclosed herein.

Shatter resistant capsules were characterized in fully developed capsules having at most 10% seed moisture, and the shatter-resistant capsules were characterized by at least one of the features: (i) at least 80% seed retention after shaking the plant, (ii) at least 80% seed retention after the capsules are turned upside down, (iii) a ratio of at least 5:1 between a total length of the capsule and a length of a zone in which the capsule tips are open, and/or 20-30% of the capsules retain 90-95% of the seeds in fully developed green capsules before drying.

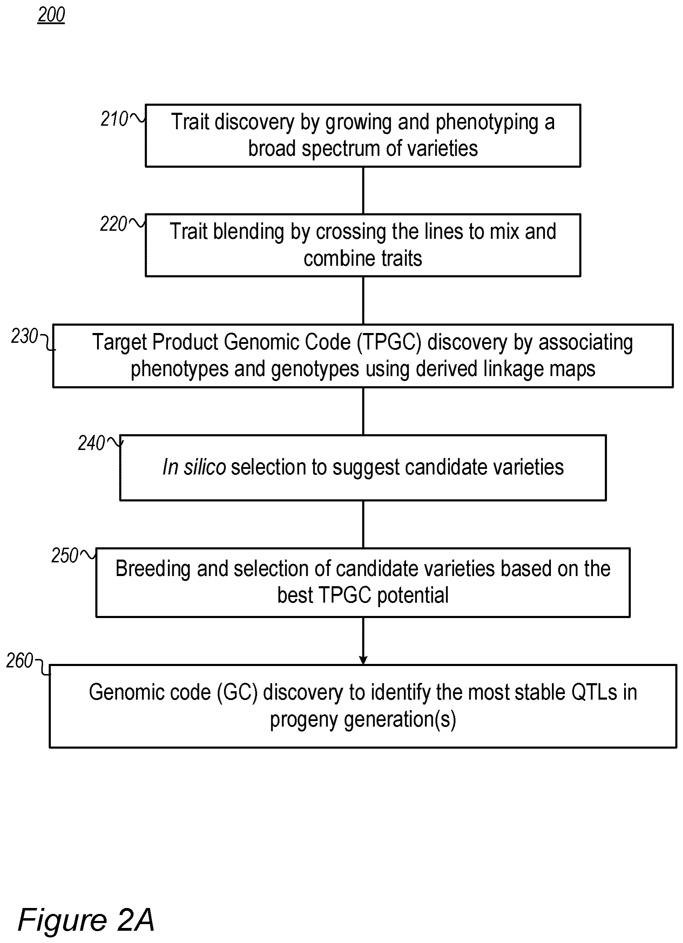

Various embodiments comprise sesame plants with shatter-resistant capsules, or part(s) thereof, that comprise a plurality of loci associated with a corresponding plurality of quantitative trait loci (QTLs) having a corresponding plurality of nucleic acid genetic markers that are associated with a plurality of phenotypic traits of the sesame plant. The QTLs are combined in the sesame plants from a plurality of sesame varieties using computationally supported breeding tools. Phenotypic and genotypic analyses of many sesame varieties were performed to derive markers for phenotypic traits that contribute to improved taste, high protein and/or to specific amino acid composition, and a breeding simulation was used to identify the most common and most stable markers. Examples of such phenotypic traits include the protein content, amino acid composition, e.g., with respect to fractions of specific amino acids such as methionine, cysteine, phenylalanine etc., and taste traits evaluated semi-quantitatively in tasting tests. Following verification of trait stability over several generations, markers and marker cassettes were defined as being uniquely present in the developed sesame lines. The resulting high protein sesame lines can be used to increase sesame product quality and enable combining sesame products with products of other plants to yield various textured vegetable protein (TVP) products with required composition that may not be available from the other plants. For example, high protein sesame may be used to enhance the protein level of other plant products such as pea concentrates - to yield products with specified nutritional values, e.g., as meat replacements. Details concerning the QTLs and markers are provided in **Table 1** below, and the methods used to develop and select the varieties are disclosed with respect to **Figure 2A** below.

Moreover, various sesame products were tested and characterized with respect to trait improvements provided by the disclosed varieties. Examples for sesame products include seeds and dehulled seeds for various uses (e.g., baked and fried goods), tahini spreads and other spreads, dips and sauces based on tahini, halva condiments, protein and sports bars, granola mixes, alternative milk products, confectionery, etc.

It is noted that disclosed sesame plants with shatter-resistant capsules are hybridized and that none of the disclosed varieties occur in nature or in known worldwide sesame varieties. The sesame plants with shatter-resistant capsules are characterized by the disclosed QTL markers which were judiciously detected in other varieties, selected and gradually introduced in the disclosed combinations to yield the disclosed sesame plants with shatter-resistant capsules. Once specific disclosed sesame plants with shatter-resistant capsules were achieved, further breeding was used to stabilize the varieties and assure constant phenotypes for sesame production, making the varieties pure lines. The terms "hybridized" or "introgressed" are used herein to define disclosed varieties having QTL markers and traits collected during the breeding process from different varieties of sesame that were determined and hybridized during the highly complicated computationally-supported breeding methods described below, in which the genotypes of multiple sesame varieties have been judiciously combined and analyzed, to discover and accumulate the recited QTL markers and corresponding phenotypical traits into the disclosed sesame plant with shatter-resistant capsules. Although the recited sesame plants are not genetically modified by sequences originating from other species, they cannot be reached merely by natural processes, as is evident by the detailed and intentional breeding program that was applied to specifically measure required characteristics, detect corresponding markers using bioinformatics methods and combine the detected QTLs in the selected varieties by classic breeding approaches (e.g., hand pollination crosses and single plant selections). Further generations derived from the disclosed sesame plants with shatter-resistant capsules may also be understood to be a sesame plant with shatter-resistant capsules.

As described herein, six unique combinations of QTLs, referred to as QTL cassettes, were detected to differentiate disclosed breeding material (with shattering resistant capsules) from worldwide sesame lines. The cassettes' discovery was based on ca. 600 elite shatter resistant lines (disclosed, e.g., in U.S. Patent No. 10,577,623) and a set of ca. 200 world accessions. Following the discovery of the cassettes, the disclosed germplasm was tested with respect to the world accessions to demonstrate their uniqueness, as being clearly distinct from known sesame varieties (see **Table 5).** The six cassettes share one common marker (QTL1) which is associated with the taste evaluation of the sesame seeds. Moreover, other markers (QTLs 2-10) in the cassettes were found to be associated with various protein content, amino acid composition and taste components.

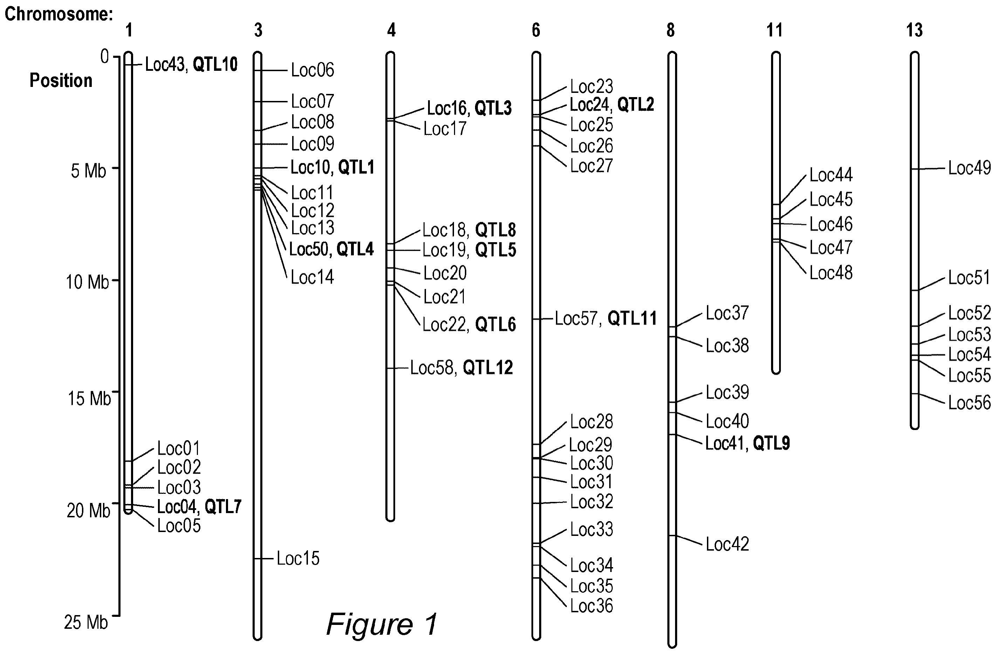

**Figure 1** is a high-level schematic illustration of sesame chromosomes with indications of the relevant markers' loci, according to some embodiments of the invention. **Figure 1** illustrates schematically seven of the thirteen sesame chromosomes and the marker locations indicated along them. **Table 3** below lists all loci, their markers and their respective traits. The details of cassette composition are provided below. The chromosomal position refers to the Genome V2.0 version (Wang et al. 2016, Updated sesame genome assembly and fine mapping of plant height and seed coat color QTLs using a new high-density genetic map, BMC Genomics 17:31).

**Table 1** provides the derived genetic markers, QTLs, corresponding traits and resulting marker cassettes, according to some embodiments of the invention. Protein, ash, carbohydrates and amino acids levels were measured by using Perten Instruments DA 7200 NIR analysis system, calibrated for sesame seeds. Protein, ash and carbohydrates content were measured as gr per 100gr sesame seeds (percent as-is), while the amino acid content was measured as amino acid content (gr/100gr) relative to the protein level - expressing the amino acid fraction from the protein content, to address the correlation of amino acid level with the overall protein content. Taste components (General taste grade and bitterness) were measured by physical tasting of at least five tasters per test. For each taste component, tasters scored the specific seeds varieties with a relative score of taste and bitterness. Tastiness was evaluated semi-quantitatively on a scale ranging from 1 to 9, with 1 indicating the least tasty seeds with respect to a reference variety and 9 indicating the tastiest seeds compared to the reference variety. Bitterness was evaluated semi-quantitatively on a scale ranging from 1 to 9, with 1 denoting seeds that are least, or not bitter compared to the reference variety and 9 denoting the most, or very bitter seed compared to the reference variety. The reference variety, the Ethiopian line "Humera", was scored as 4 for each component.

**Table 1: Genetic markers, QTLs, corresponding traits and marker cassettes with corresponding high protein level, enhanced amino acid content and/or improved taste components.**

| **QTL** | **SEQ ID NO** | **Chromosome-Position** | **QTL P-value** | **Phenotypic trait** |
|---|---|---|---|---|
| 1 | 1,2 | Chr 3-4959888 | 0.0084 | Taste |
| 2 | 3,4 | Chr 6-2595046 | Ash - 0.005 | Ash (low content, less bitter seeds), and |
| | | | Protein - 0.000005 | Protein level |
| 3 | 5,6 | Chr 4-2777176 | 0.00005 | Methionine |
| 4 | 7,8 | Chr 13-5809997 | 0.00007 | Methionine, Phenylalanine |
| 5 | 9,10 | Chr 4-8636698 | 0.0002 | Methionine |
| 6 | 11,12 | Chr 4-10189573 | Carbohydrates - 0.0006 | Carbohydrates (lower level, higher complementary protein and oil), and |
| | | | Protein - 0.0003 | Protein level |
| 7 | 13,14 | Chr 1-20018096 | 0.0005 | Methionine |
| 8 | 15, 16 | Chr 4-8384304 | 0.001 | Protein level |
| 9 | 17, 18 | Chr 8-16918017 | 0.0008 | Protein level |
| 10 | 19, 20 | Chr 11-304411 | 0.0008 | Taste |
| 11 | 21, 22 | Chr 6-11699161 | 0.013 | Protein level |
| 12 | 23, 24 | Chr 3-13967471 | 0.0001 | Protein level |

**Table 1 (continued):**

| **QTL** | **SEQ ID NO** | **Allele** | | **Cassettes (with respective QTLs)** | | | | | | |
|---|---|---|---|---|---|---|---|---|---|---|
| | | **1** | **2** | **1** | **2** | **3** | **4** | **5** | **6** | **7** |
| 1 | 1,2 | A | G | AA/AG | AA/AG | AA/AG | AA/AG | AA/AG | AA/AG | AA/AG |
| 2 | 3,4 | A | G | AA/AG | AA/AG | AA/AG | AA/AG | | | |
| 3 | 5,6 | A | G | AA/AG | AA/AG | GG/GA | GG/GA | | | |
| 4 | 7,8 | A | G | AA/AG | GG/GA | | | | | |
| 5 | 9,10 | C | T | | | CC/CT | TT/TC | | | |
| 6 | 11,12 | A | G | | | | AA/AG | | | |
| 7 | 13, 14 | T | C | | TT/TC | | | | | |
| 8 | 15, 16 | T | C | | | | | TT/TC | | |
| 9 | 17, 18 | G | A | | | | | GG/GA | | |
| 10 | 19, 20 | T | C | | | | | | TT/TC | |
| 11 | 21, 22 | T | C | | | | | | | TT/TC |
| 12 | 23, 24 | C | T | | | | | | | CC/CT |

Disclosed sesame plants with shatter-resistant capsules were derived by computationally supported breeding methods to yield plants which are different and distinct from any prior art sesame varieties. Specifically, disclosed sesame plants have QTL 1 in common and are grouped herein by combinations of QTLs denoted in **Table 1** as cassettes 1 through cassette 7.

**Tables 2** and **3** present all analyzed genetic markers (including QTLs 1-10) with their respective discovered phenotypic traits, according to some embodiments of the invention. The traits are numbered 1-16 and are related to loci numbered 1-56 of the sesame genome. **Table 2** is ordered roughly according to the order of the QTLs; **Table 3** is ordered by locus number with respect to the chromosomal position of the loci, as illustrated schematically in **Figure** 1. The phenotypic traits were measured as described above concerning **Table 1.**

**Table 2: Phenotypic traits that relate to high protein content, amino acid fractions in protein composition, ash, carbohydrates and taste components - and their respective markers' loci.**

| **Trait** | **Description** | **Marker loci associated with the trait (see Table 3)** |
|---|---|---|
| Tr16 | Taste general sensory grade | Loc10 - **QTL 1,** Loc43 - **QTL10** |
| Tr11 | Protein level | Loc15, Loc18 - **QTL 8,** Loc20, Loc22 - **QTL 6,** Loc24 **-QTL 2,** Loc26, Loc29, Loc41 - **QTL 9,** Loc42, Loc44, **Loc57-QTL11,** Loc58**-QTL12** |
| Tr08 | Methionine fraction | Loc04 - **QTL 7,** Loc09, Loc16 - **QTL 3,** Loc19 - **QTL 5,** Loc25, Loc27, Loc28, Loc50 - **QTL 4** |
| Tr01 | Alanine fraction | Loc07, Loc32 |
| Tr02 | Arginine fraction | Loc01, Loc02, Loc04 - **QTL 7,** Loc05, Loc11, Loc21, Loc30, Loc39, Loc40, Loc51 |
| Tr03 | Glutamic acid fraction | Loc45, Loc52 |
| Tr04 | Glycine fraction | Loc07, Loc30, Loc33, Loc35, Loc36, Loc54, Loc55 |
| Tr05 | Histidine fraction | Loc09, Loc32, Loc49, Loc54 |
| Tr06 | Isoleucine fraction | Loc04 - **QTL 7,** Loc09, Loc17, Loc52 |
| Tr07 | Leucine fraction | Loc01, Loc04 - **QTL 7,** Loc05, Loc09, Loc12, Loc33, Loc35, Loc40, Loc48, Loc53, Loc54 |
| Tr09 | Phenylalanine fraction | Loc01, Loc03, Loc04 - **QTL 7,** Loc05, Loc06, Loc08, Loc09, Loc34, Loc50 - **QTL 4,** Loc51, Loc52 |
| Tr10 | Proline fraction | Loc06, Loc07, Loc31, Loc33, Loc35, Loc36, Loc37, Loc38, Loc53, Loc54, Loc56 |
| Tr12 | Serine fraction | Loc03, Loc04 - **QTL 7,** Loc13, Loc34, Loc35, Loc40, Loc46, Loc47, Loc51, Loc53 |
| Tr13 | Threonine fraction | Loc04 - **QTL 7,** Loc06, Loc09, Loc14, Loc23, Loc32, Loc36, Loc54 |
| Tr14 | Tyrosine fraction | Loc04 - **QTL 7,** Loc05, Loc09 |
| Tr15 | Valine fraction | Loc01, Loc04 - **QTL 7,** Loc05, Loc08, Loc09, Loc13, Loc36, Loc50 - **QTL 4,** Loc53, Loc54 |
| Tr17 | Carbohydrates level | **QTL 6,** Loc15, Loc53 |
| Tr18 | Ash content | Loc01, **QTL 2,** Loc29, Loc41 |

It is noted that while some of the genetic markers disclosed herein have been identified in previous applications (e.g., locus 9 disclosed herein is similar to QTL 5 of U.S. Patent No. 11,044,884, locus 35 disclosed herein is similar to locus 33 in U.S. Application No. 17/474,944 and to QTL 3 of U.S. Patent No. 10,577,623 and loci 1, 3, 4 (QTL 7), 24 (QTL 2), 28, 29, 31, 33, 34, 36, 38, 50 (QTL 4) and 55 are similar to loci 8, 9, 10, 21, 25, 26, 31, 32, 34, 35, 60 and 63, respectively, U.S. Application No. 17/474,944) - none of the previous applications teaches the association of the respective loci with any of the currently disclosed traits of protein content, amino acid content and/or taste properties.

**Table 3: Analyzed genetic markers (including QTLs 1-9), their loci and their respective traits.**

| **Marker loci and QTLs** | **Chromosome - Position** | **Traits** |
|---|---|---|
| Loc01 | Chr 1 - 18095873 | Tr02, Tr07, Tr09, Tr15, Tr18 |
| Loc02 | Chr 1 - 19182237 | Tr02 |
| Loc03 | Chr 1 - 19270856 | Tr09, Tr12 |
| Loc04, QTL07 | Chr 1 - 20018096 | Tr06, Tr08, Tr09, Tr13, Tr14, Tr02, Tr07, Tr12, Tr15 |
| Loc05 | Chr 1 - 20180505 | Tr02, Tr07, Tr14, Tr15, Tr09 |
| Loc06 | Chr 3 - 588685 | Tr09, Tr10, Tr13 |
| Loc07 | Chr 3 - 1987260 | Tr01, Tr04, Tr10 |
| Loc08 | Chr 3 - 3292055 | Tr09, Tr15 |
| Loc09 | Chr 3 - 3895479 | Tr05, Tr06, Tr07, Tr08, Tr09, Tr13, Tr14, Tr15 |
| Loc10, QTL01 | Chr 3 - 4959888 | Tr16 |
| Loc11 | Chr 3 - 5304519 | Tr02 |
| Loc12 | Chr 3 - 5450856 | Tr07 |
| Loc13 | Chr 3 - 5666431 | Tr12, Tr15 |
| Loc14 | Chr 3 - 5957188 | Tr13 |
| Loc15 | Chr 3 - 22470954 | Tr11, Tr17 |
| Loc16, QTL03 | Chr 4 - 2777176 | Tr08 |
| Loc17 | Chr 4 - 2850209 | Tr06 |
| Loc18, QTL08 | Chr 4 - 8384304 | Tr11 |
| Loc19, QTL05 | Chr 4 - 8636698 | Tr08 |
| Loc20 | Chr 4 - 9433829 | Tr11 |
| Loc21 | Chr 4 - 10067129 | Tr02 |
| Loc22, QTL06 | Chr 4 - 10189573 | Tr11, Tr17 |
| Loc23 | Chr 6 - 1954272 | Tr13 |
| Loc24, QTL02 | Chr 6 - 2595046 | Tr11 |
| Loc25 | Chr 6 - 2672215 | Tr08 |
| Loc26 | Chr 6 - 3265625 | Tr11 |
| Loc27 | Chr 6 - 3998267 | Tr08 |
| Loc28 | Chr 6 - 17339135 | Tr08 |
| Loc29 | Chr 6 - 17949954 | Tr11, Tr18 |
| Loc30 | Chr 6 - 17964493 | Tr02, Tr04 |
| Loc31 | Chr 6 - 18811179 | Tr10 |
| Loc32 | Chr 6 - 19981666 | Tr01, Tr05, Tr13 |
| Loc33 | Chr 6 - 21794639 | Tr04, Tr10, Tr07 |
| Loc34 | Chr 6 - 21918155 | Tr09, Tr12 |
| Loc35 | Chr 6 - 22739924 | Tr04, Tr10, Tr07, Tr12 |
| Loc36 | Chr 6 - 23288849 | Tr04, Tr10, Tr13, Tr15 |
| Loc37 | Chr 8 - 12066296 | Tr10 |
| Loc38 | Chr 8 - 12548680 | Tr10 |
| Loc39 | Chr 8 - 15457227 | Tr02 |
| Loc40 | Chr 8 - 15924945 | Tr02, Tr07, Tr12 |
| Loc41, QTL09 | Chr 8 - 16918017 | Tr11, Tr18 |
| Loc42 | Chr 8 - 21427508 | Tr11 |
| Loc43, QTL10 | Chr 11 - 304411 | Tr16 |
| Loc44 | Chr 11 - 6543963 | Tr11 |
| Loc45 | Chr 11 - 7227477 | Tr03 |
| Loc46 | Chr 11 - 7433357 | Tr12 |
| Loc47 | Chr 11 - 8149740 | Tr12 |
| Loc48 | Chr 11 - 8273970 | Tr07 |
| Loc49 | Chr 13 - 4983980 | Tr05 |
| Loc50, QTL04 | Chr 13 - 5809997 | Tr09, Tr15, Tr08 |
| Loc51 | Chr 13 - 10447577 | Tr02, Tr09, Tr12 |
| Loc52 | Chr 13 - 12034281 | Tr03, Tr06, Tr09 |
| Loc53 | Chr 13 - 12882168 | Tr12, Tr07, Tr10, Tr15, Tr17 |
| Loc54 | Chr 13 - 13370733 | Tr04, Tr05, Tr07, Tr10, Tr13, Tr15 |
| Loc55 | Chr 13 - 13555490 | Tr04 |
| Loc56 | Chr 13 - 15093301 | Tr10 |
| Loc57, QTL11 | Chr 6 - 11699161 | Tr11 |
| Loc58, QTL12 | Chr 3 - 13967471 | Tr11 |

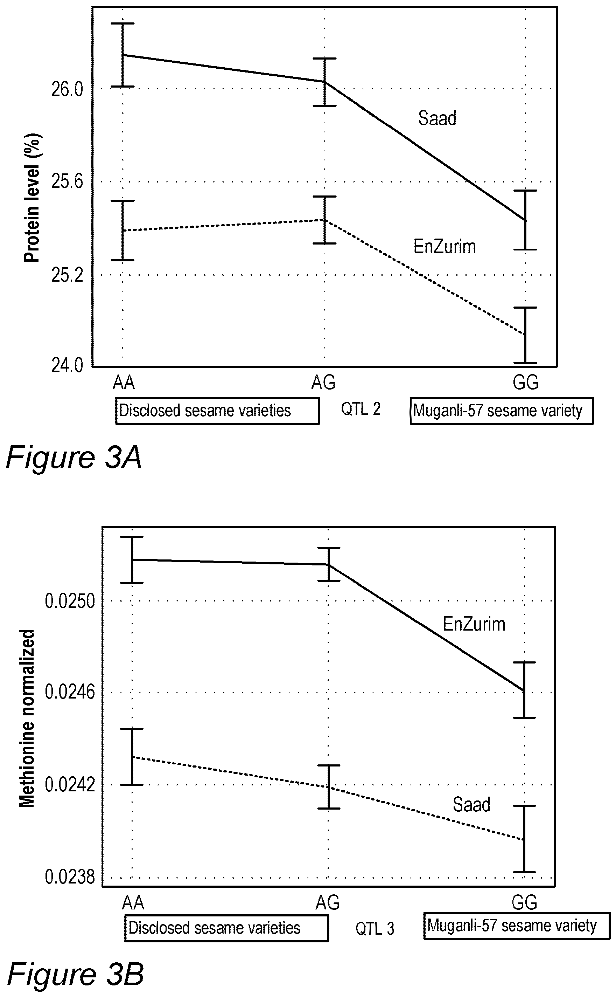

**Figure 3A** illustrates the effects of the marker states of QTL 2 on the protein level in disclosed varieties, according to some embodiments of the invention. The state of QTL 2 (homozygous SEQ ID NO 3 marked as AA, homozygous SEQ ID NO 4 in Muganli-57 sesame variety marked as GG, and heterozygosity marked as AG), was correlated with the protein content at two different environments (marked Saad and EnZurim) - indicating enhanced protein content correlating with the state of QTL 2 in both environments (e.g., 26.2% for homozygous SEQ ID NO 3 versus 25.3% for homozygous SEQ ID NO 4 at Saad [p-value: 0.0001] and 25.2% for homozygous SEQ ID NO 3 versus 24.9% for homozygous SEQ ID NO 4 at EnZurim [not significant]).

**Figure 3B** illustrates the effects of the marker states of QTL 3 on the methionine level in disclosed varieties, according to some embodiments of the invention. The state of QTL 3 (homozygous SEQ ID NO 5 marked as AA, homozygous SEQ ID NO 6 in Muganli-57 sesame variety marked as GG, and heterozygosity marked as AG), was correlated with the methionine content at two environments (Saad and EnZurim as in **Figure 3B****)** - indicating enhanced methionine content correlating with the state of QTL 3 in both environments (e.g., 0.0252 for homozygous SEQ ID NO 5 versus 0.0246 for homozygous SEQ ID NO 6 at EnZurim [p-value: 0.002] and 0.0243 for homozygous SEQ ID NO 5 versus 0.240 for homozygous SEQ ID NO 6 at Saad[p-value 0.062]).

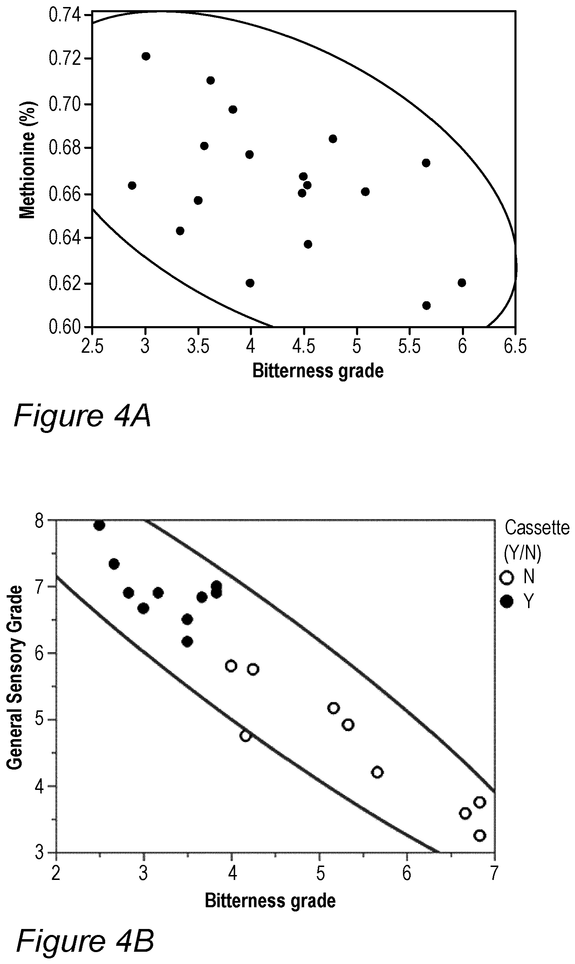

**Figures 4A-4D** illustrate the relation between several parameters of the seeds that were found to correlate with bitterness, according to some embodiments of the invention. It is noted that the correlations presented in **Figures 4A-4D** were detected three different and independent data sets which reflect three breeding cycles, each carried out in several environments.

**Figure 4A** illustrates the relation between the methionine level and bitterness, indicating a negative correlation between methionine level and bitter taste in two independent datasets (sesame grown in years 2017 and 2018, respectively) - by providing a bivariate fit of methionine percentage to the bitterness grade (the ellipses indicate p values of 0.95, the correlation factor being -0.5, with p <0.05). Accordingly, the inventors note that by increasing methionine level through the selection of varieties with the respective QTLs for high methionine fractions (e.g., QTLs 3, 4, 5 and 7), the taste of the resulting sesame may be improved, in addition to directly selecting varieties with QTLs for improved taste (such as QTLs 1 and 10).

**Figure 4B** illustrates the relation between the general sensory grade and the bitterness grade by providing a bivariate fit of the general sensory grade to the bitterness grade (the ellipses indicate p values of 0.95, the correlation factor being -0.95 with p <0.0001). Accordingly, the inventors note that the strong correlation indicates that by decreasing the bitterness level (due to several factors) the general sensory grade is improved directly. Hence, additional factors have been evaluated with respect to the bitterness grade (which strongly correlates with the general sensory grade). It is noted that data for seeds including any of the marker cassettes (indicated as cassette - Y in the figure) score significantly higher on the general sensory grade and lower on the bitterness scale (by ca. 1-4 grades on each scale) compared to prior art seeds (including the Muganli-57 and Humera varieties).

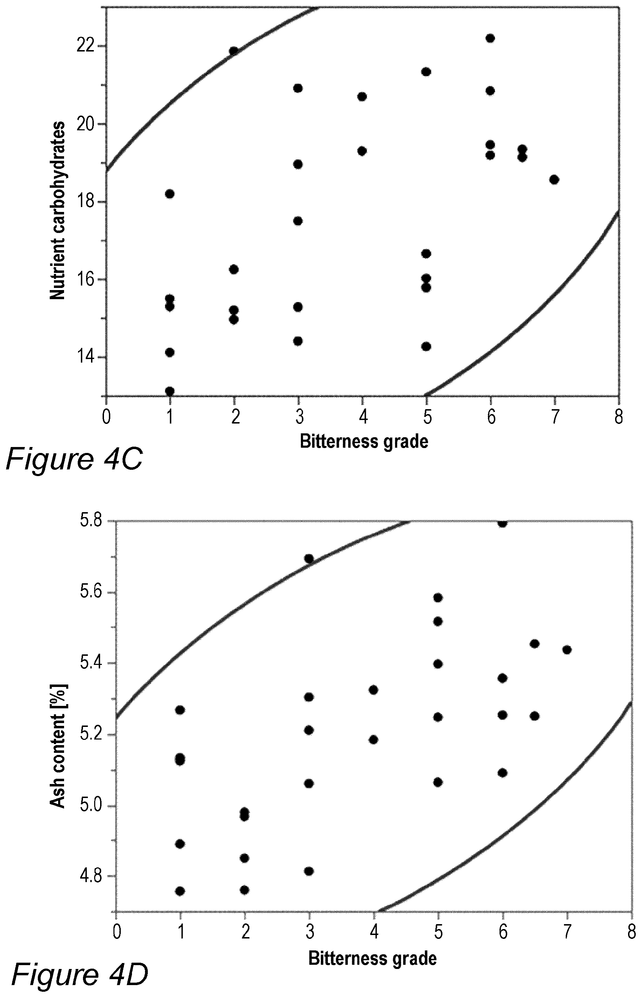

**Figures 4C** and **4D** illustrate the correlation of two such factors, namely the levels of carbohydrates and ash, respectively, with the bitterness grade. Carbohydrates and ash content was measured by NOR, The resulting correlations are clear and significant (ANOVA bivariate fit, the ellipses indicate p values of 0.95, the correlation factors being 0.49, with p=0.0075 for carbohydrates content - **Figure 4C****;** and 0.59, with p=0.0009 for ash content - **Figure 4D****),** and indicate that markers associate with low content of ash and carbohydrates in the seeds (e.g., QTLs 2 and 6, respectively) also contribute to improved taste. It is noted that these data are accumulated from several growth cycles and years during the breeding program (irrespective of the specific genetic composition).

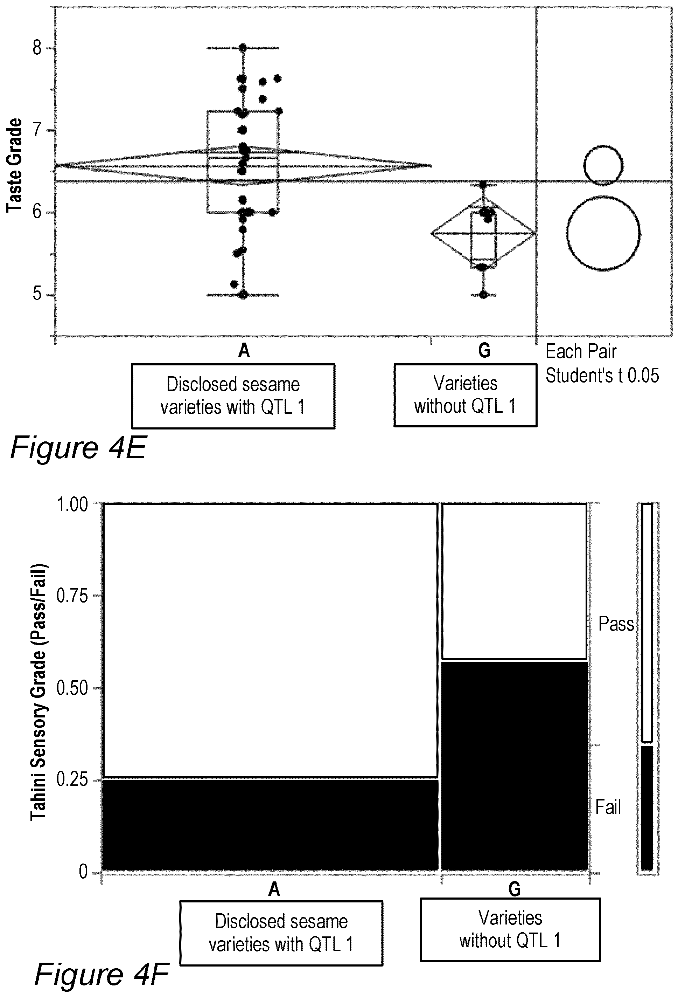

**Figures 4E** and **4F** illustrate the corresponding effect of QTL 1 on the taste of disclosed varieties (taste of seeds in **Figure 4E** and taste of tahini prepared from the seeds in **Figure 4F****),** according to some embodiments of the invention. **Figure 4E** provides a correlation between the taste grade and the state of QTL 1 (homozygous to SEQ ID NO 1 in disclosed varieties versus the wildtype which is homozygous to SEQ ID NO 2) - indicating a significant difference between an average taste grade of 6.5 for disclosed varieties versus an average taste grade of 6.0 for Muganli-57 sesame variety (applying Student's t test with a significance level of 0.05). It is noted that both the Muganli-57 sesame variety with the G allele at QTL 1 and disclosed varieties with the A allele at QTL 1 have improved taste with respect to a common commercial variety, the Ethiopian line "Humera", which scored as 4 in the taste scale.

As illustrated in **Figure 4F****,** disclosed varieties with QTL 1 homozygous to SEQ ID NO 1 also have a higher rate of success in a tasting test performed with tahini prepared from the respective seeds - compared with seeds of Muganli-57 sesame variety (success rate of 75% for the disclosed varieties, compared with 40-45% for the Muganli-57 sesame variety). While subjective, the tasting test yielded statistically significant improvement (p=0.0413) in taste for tahini made of disclosed sesame varieties.

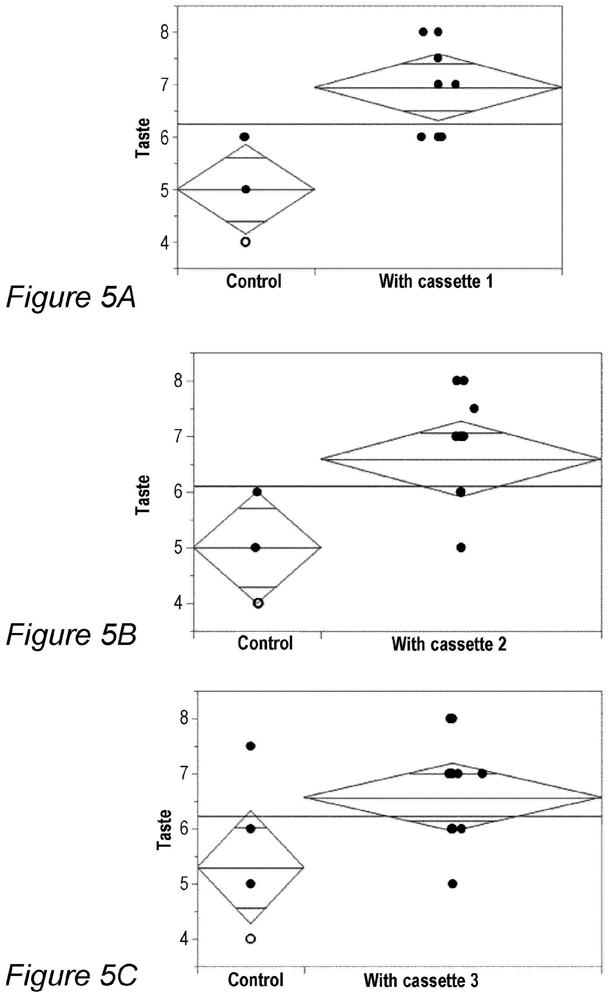

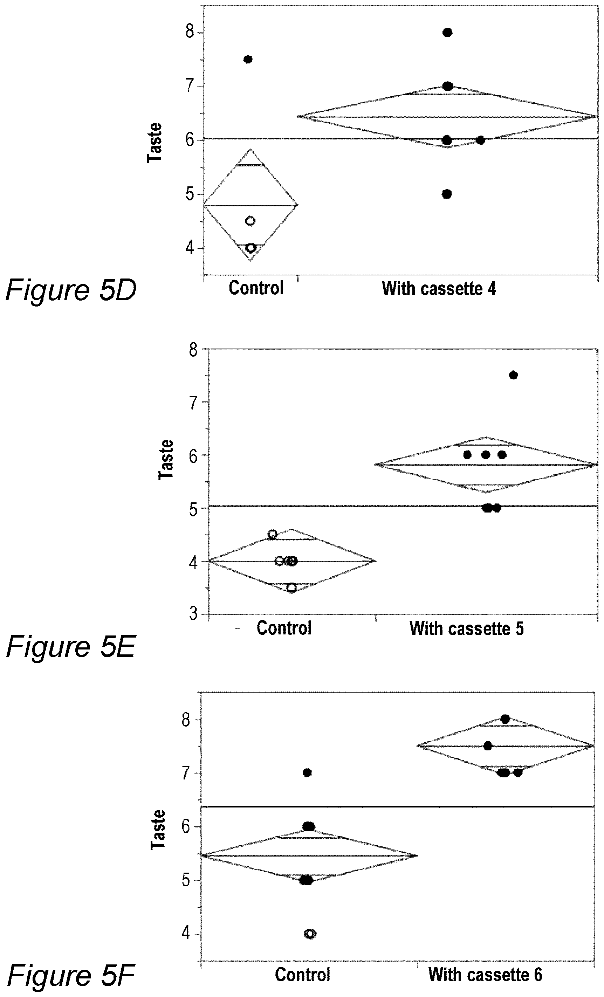

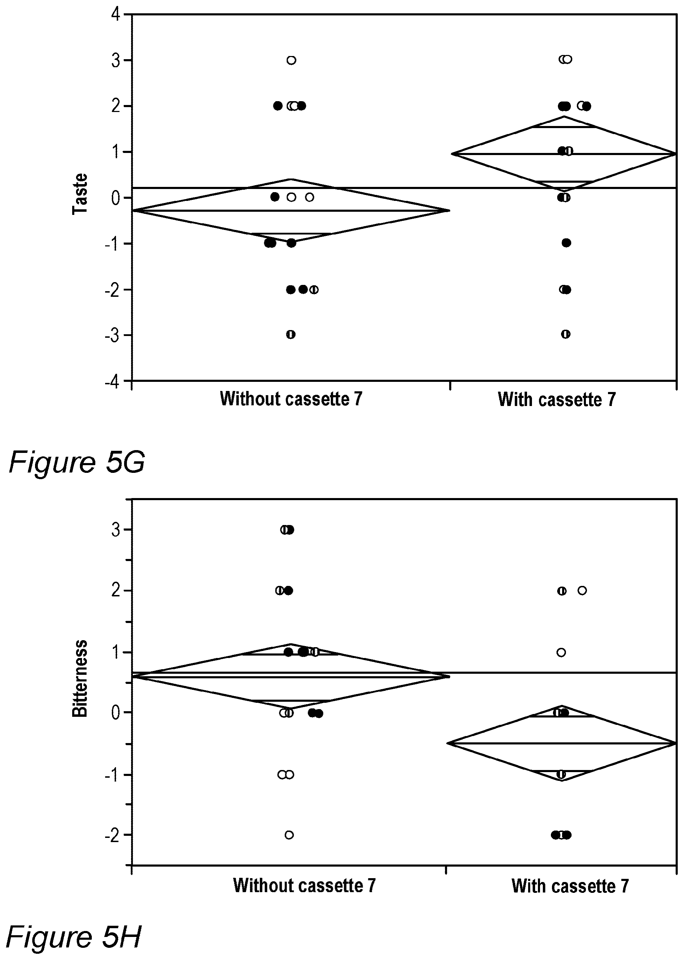

**Figures 5A-5H** illustrate the improved taste of seeds from disclosed varieties with each of the marker cassettes, according to some embodiments of the invention. The initial data provides comparisons of seeds from a few of the disclosed lines having each marker cassette compared with prior art line Muganli-57 sesame variety as the controls (grown under the same conditions). **Table 4** (below) provides one-way analysis of variance (ANOVA) results indicating the taste improvement with respect to the prior art, which are significant for cassettes 3, 4 and 5 (p values 0.039, 0.0094 and 0.0003 respectively; p values for cassettes 1, 2 and 6 were 0.0018, 0.0139 and <0.0001, respectively). For sesame lines having Cassette 7 taste and bitterness comparison were made with respect to prior art Humera variety and the comparison included additional ten samples - five from lines including cassette 7 and five from prior art line not including cassette 7. Samples were randomized and are presented with respect to the results for the Humera line (allocated the zero value) using a scale between +3 for best tasting and -3 for least tasty **(****Figure 5G****)** and between +3 for very bitter and -3 for least bitter **(****Figure 5H****).** The results show there is a significant effect on taste and bitterness for varieties that comprise cassette 7 markers, with a line denoted ES309 (including cassette 7 markers) scored as the best variety in term of general taste and bitterness. The effect of cassette 7 was measured as adding 1.24 on the taste scale (p=0.013, significant) and reducing bitterness by 1.1 on the bitterness scale (p=0.005, significant). Different hatching/shade designate different testers. It is noted that all comparisons refer to disclosed varieties versus prior art varieties, that were grown under the same conditions.

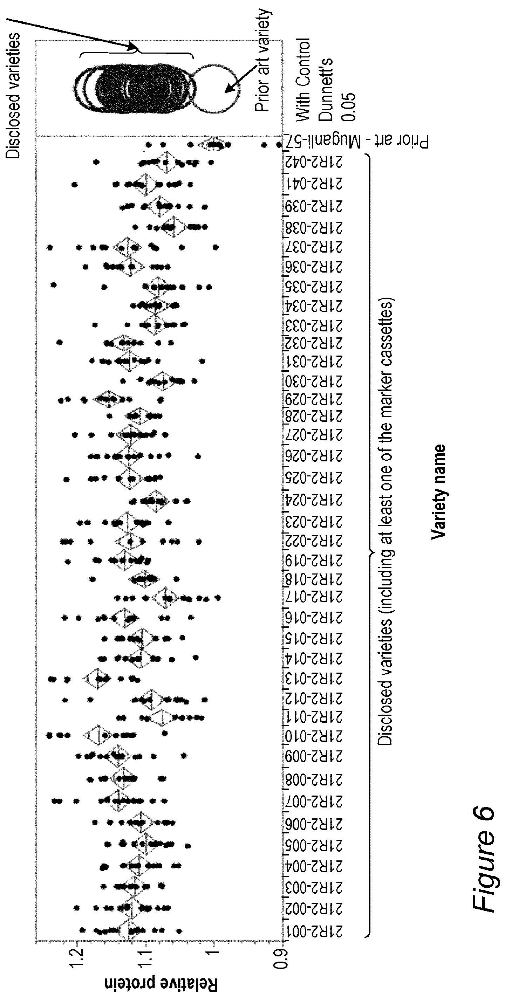

**Figure 6** illustrates the relative protein content of disclosed sesame lines compared to the Muganli-57 sesame variety, according to some embodiments of the invention. The variability is related to different fields and growing conditions - which correspondingly result in different results for the protein content of the seeds. Still, averaged over the range of variety resulting from the growing conditions, **Figure 6** indicates that disclosed sesame varieties including the disclosed markers yield a relative increase of 10% in average in protein content compared to the Muganli-57 sesame variety.

**Table 4** provides the average effects of sesame varieties with different cassette combinations on the protein content and on the taste grades of the respective varieties, compared to lines not expressing any of the cassettes (specifically the Muganli-57 sesame variety, grown under the same conditions). It is noted that as the content of nutrients in the seeds depends on the growing environment, using the Muganli-57 sesame variety for comparison under similar conditions provides a reliable reference for the improvements achieved by the disclosed sesame varieties. **Table 4** thus provides non-limiting examples for the improved performance of some of the disclosed varieties. It is noted that the values are averaged over multiple lines grown under different environmental conditions, and that specific lines with the disclosed cassettes may be selected to improve protein levels, e.g., by 5-10% compared to the Muganli-57 sesame variety (see, e.g., **Figure 6****),** as well as to improve taste, e.g., by at least one step of the evaluation scale compared to the Muganli-57 sesame variety (see, e.g., **Figures 5A-5F****).** It is noted that taste was improved by modifying several parameters such as the levels of ash, carbohydrates and methionine, as illustrated herein (see, e.g., **Figures 4A-4D****).** It is further noted that lines with specific combinations of cassettes may be selected to provide specific increases, in protein content and/or in parameters relating to taste with respect to sesame varieties lacking the disclosed cassettes.

**Table 4:** Protein content and taste enhancements of sesame varieties with different cassette combinations, compared to lines not expressing any of the cassettes.

| Cassette | Relative protein content | Improved taste score (vs. prior art varieties) |
|---|---|---|
| 1 | +5.8% | +1.94 (6.94 vs. 5.0) |
| 2 | +5.3% | +1.59 (6.59 vs. 5.0) |
| 3 | +5.1% | +1.27 (6.57 vs. 5.3) |
| 4 | +6.3% | +1.63 (6.43 vs. 4.8) |
| 5 | +7% | +1.81 (5.81 vs. 4.0) |
| 6 | +0.9% | +2.05 (7.5 vs. 5.45) |
| 7 | +1.2% | +1.24 |

It is noted that the cassettes provide combinations of specific QTLs that are associated with specific traits, e.g., cassettes 1, 2 and 3 include QTLs 3, 4, 5 and 7 (see **Table 1** for the exact combinations) associated with high methionine levels and reduced bitterness (improved taste), cassettes 1, 2, 3, 4 and 5 include QTLs 2, 6, 8 and 9 associated with high protein levels, and cassettes 1-6 include QTLs 1-7 and 10 associated directly or indirectly (via increased methionine, reduced ash or reduced carbohydrates) with better taste and reduced bitterness.

**Table 5** illustrates the uniqueness of the disclosed varieties, as characterized by cassettes 1-6 and their combinations, according to some embodiments of the invention, with respect to the world varieties. The number of disclosed varieties is indicated with respect to the combination of marker cassettes which characterize the respective varieties.

**Table 5: Comparison of disclosed varieties included in the disclosed cassettes with world (prior art) varieties.**

| **Cassette combinations** | | | | | | | **Sesame lines** | |
|---|---|---|---|---|---|---|---|---|
| 1 | 2 | 3 | 4 | 5 | 6 | 7 | Disclosed (out of 600) | World (out of 200) |
| yes | yes | | | yes | yes | yes | 1 | 0 |
| yes | | yes | yes | yes | yes | yes | 1 | 0 |
| yes | | | | yes | yes | | 1 | 0 |
| | | yes | yes | yes | yes | | 1 | 0 |
| | | | yes | yes | yes | | 2 | 0 |
| | | | | yes | yes | | 8 | 0 |
| yes | | yes | | | yes | | 1 | 0 |
| yes | | | | | yes | | 1 | 0 |
| | yes | | | | yes | | 1 | 0 |
| | | yes | | | yes | yes | 3 | 0 |
| | | | yes | | yes | | 1 | 0 |
| | | | | | yes | | 8 | 0 |
| yes | yes | yes | yes | yes | | | 6 | 0 |
| yes | yes | yes | | yes | | | 1 | 0 |
| yes | yes | | yes | yes | | | 3 | 0 |
| yes | yes | | | yes | | | 10 | 0 |
| yes | | yes | yes | yes | | | 6 | 0 |
| yes | | | yes | yes | | | 4 | 0 |
| yes | | | | yes | | | 17 | 0 |
| | yes | yes | yes | yes | | | 5 | 0 |
| | yes | | yes | yes | | | 1 | 0 |
| | yes | | | yes | | | 6 | 0 |
| | | yes | yes | yes | | | 6 | 0 |
| | | yes | | yes | | | 3 | 0 |
| | | | yes | yes | | | 12 | 0 |
| | | | | yes | | | 108 | 0 |
| yes | yes | yes | yes | | | | 2 | 0 |
| yes | yes | | | | | | 4 | 0 |
| yes | | yes | yes | | | | 8 | 0 |
| yes | | yes | | | | | 4 | 0 |
| yes | | | yes | | | | 2 | 0 |
| yes | | | | | | | 13 | 0 |
| | yes | yes | yes | | | | 3 | 0 |
| | yes | yes | | | | | 1 | 0 |
| | yes | | | | | | 8 | 0 |
| | | yes | yes | | | | 7 | 0 |
| | | yes | | | | yes | 63 | 0 |
| | | | yes | | | | 10 | 0 |
| | | | | | | yes | 1 | 0 |

A total of 281 varieties are thus shown to be included in one of the combinations of disclosed cassettes, while none of the world varieties that were examined includes any of the cassette combinations. All disclosed varieties are shatter resistant and characterized by improved taste, increased protein content and/or improved taste (e.g., with increased methionine content and/or reduced ash or carbohydrates content).

Advantageously, disclosed embodiments provide sesame plants with high protein that may improve the nutritional value of the sesame seeds, e.g., with respect to protein content and/or with respect to the nutritional value related to specific amino acids (see **Table 2).** These varieties and products therefrom (e.g., isolates and/or concentrates) may be used to enrich products made from most legume and cereal crops, such as pea, soybean, wheat, barley, rice, corn and others that are low in sulfur-containing amino acids such as methionine and cysteine. Accordingly, disclosed sesame plants and products thereof may be used as complementary supplements to cereal and legume proteins.

As used herein, sesame plants with high protein content provide an improvement of at least a 5% (e.g., typically between 5% and 10%) relative increase in protein content compared to the Muganli-57 sesame variety not expressing any of the marker cassettes described herein (and grown under the same conditions). Alternatively or complementarily, disclosed sesame varieties have at least 10% more protein, which corresponds to (for example) a NIR value of 25%, compared with 22% for the Muganli-57 sesame variety which expressed a relative increase of 13% more protein (grown under the same conditions). In various embodiments, the protein level in disclosed sesame varieties, as measured by NIR, yields values between 24-26%.

In various embodiments, sesame plants with high methionine content and/or low ash or carbohydrate levels provide an improvement of taste and reduce bitterness compared with the Muganli-57 sesame variety not expressing any of the marker cassettes described herein (grown under the same conditions). As shown in **Table 4,** the improvement in taste is typically by one to two tasting scale grades. In various embodiments, sesame plants with improved taste are defined as providing an improvement of at least half an evaluation step in the tasting tests (see **Figures 5A-5F** and **Table 4)** compared to the Muganli-57 sesame variety which does not express any of the marker cassettes described herein (grown under the same conditions). Alternatively or complementarily, sesame plants with improved taste are defined as having a test grade of at least 6.5.

The Muganli-57 sesame variety is commonly used sesame variety in Turkey and is available from the West Mediterranean Agricultural Research Institute, a public agricultural research institute located at Demircikara Mahallesi Pasha Kavaklar 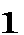 Cad. No:11 MURATPASHA / ANTALYA, Turkey. The Muganli-57 sesame variety is registered at the research institute as described in Yol et al., "A high-density SNP genetic map construction using ddRAD-Seq and mapping of capsule shattering trait in sesame," Frontiers in Plant Science, 12:679659 (2021),

QTL 1, as used herein, refers to a polymorphic genetic locus linked to a genetic marker at position 4959888 on sesame chromosome 3. The two alleles of the genetic marker at QTL 1 have the SNP bases "A" or "G", as set forth respectively in the nucleic acid sequences of SEQ ID NOs 1 and 2. In all cassettes (1 to 6), QTL 1 is homozygous for allele "A" (SEQ ID NO 1) or heterozygous (includes both SEQ ID NOs 1 and 2).
SEQ ID NO 1 (SNP base bold): 
AACCTCTAGAAGTATCTTTTGAAAGAATCCATTCAATACCTTTCCTTCATTGGCTTG
GTT A CCAATCTCCTCATTCTCTTCCTGCAGGGTCCTGCATTTTGCCATTAACATCTT
TCCCATTT
SEQ ID NO 2 (SNP base bold):

AACCTCTAGAAGTATCTTTTGAAAGAATCCATTCAATACCTTTCCTTCATTGGCTTG
GTT G CCAATCTCCTCATTCTCTTCCTGCAGGGTCCTGCATTTTGCCATTAACATCTT
TCCCATTT

QTL 2, as used herein, refers to a polymorphic genetic locus linked to a genetic marker at position 2595046 on sesame chromosome 6. The two alleles of the genetic marker at QTL 2 have the SNP bases "A" or "G", as set forth respectively in the nucleic acid sequences of SEQ ID NOs 3 and 4. In cassettes 1, 2, 3 and 4, QTL 2 is homozygous for allele "A" (SEQ ID NO 3), or heterozygous (includes both SEQ ID NOs 3 and 4).
SEQ ID NO 3 (SNP base bold): 
TACACACAACTCACTTTTGTATCTCAATACTCACATTCATATTCTTCTTACTACATAC
TTTTGGGCAACTTGATAAGGTCTAGTTCTCTAATCATCTTGT A ACGTGCTCACAAT
AGAAAACGCTGGTTGAGGCTTTTTTGGTTTTATCTTGGGGTAGTTGTGAGCCGATAG
TACCTGCAAGGAGGGAGGTCATTACGATTT
SEQ ID NO 4 (SNP base bold):

TACACACAACTCACTTTTGTATCTCAATACTCACATTCATATTCTTCTTACTACATAC
TTTTGGGCAACTTGATAAGGTCTAGTTCTCTAATCATCTTGT G ACGTGCTCACAAT
AGAAAACGCTGGTTGAGGCTTTTTTGGTTTTATCTTGGGGTAGTTGTGAGCCGATAG
TACCTGCAAGGAGGGAGGTCATTACGATTT

QTL 3, as used herein, refers to a polymorphic genetic locus linked to a genetic marker at position 2777176 on sesame chromosome 4. The two alleles of the genetic marker at QTL 3 have the SNP bases "A" or "G", as set forth respectively in the nucleic acid sequences of SEQ ID NOs 5 and 6. In cassettes 1 and 2, QTL 3 is homozygous for allele "A" (SEQ ID NO 5) or heterozygous (includes both SEQ ID NOs 5 and 6), while in cassettes 3 and 4, QTL 3 is homozygous for allele "G" (SEQ ID NO 6) or heterozygous (includes both SEQ ID NOs 5 and 6).
SEQ ID NO 5 (SNP base bold): 
ATTTATAGGGCTAAATGTGCCCCACCTTCCGGTCCACGTGGTGTCATCTCAAATCAT
AAGTTGTTAGATGATCTACGGTCTAGGGTCATCAGAGCGGGTC A GGTCGAGCGAT
TCTACTTGAGAGAGAAAGTGTTGGAATGGGTGACCAAAAAGTCAATTGGAGTGATT
ATTTGCCATTCGTCAATCTTTATTAAATAATAT
SEQ ID NO 6 (SNP base bold):

ATTTATAGGGCTAAATGTGCCCCACCTTCCGGTCCACGTGGTGTCATCTCAAATCAT
AAGTTGTTAGATGATCTACGGTCTAGGGTCATCAGAGCGGGTC G GGTCGAGCGAT
TCTACTTGAGAGAGAAAGTGTTGGAATGGGTGACCAAAAAGTCAATTGGAGTGATT
ATTTGCCATTCGTCAATCTTTATTAAATAATAT

QTL 4, as used herein, refers to a polymorphic genetic locus linked to a genetic marker at position 5809997 on sesame chromosome 13. The two alleles of the genetic marker at QTL 4 have the SNP bases "A" or "G", as set forth respectively in the nucleic acid sequences of SEQ ID NOs 7 and 8. In cassette 1, QTL 4 is homozygous for allele "A" (SEQ ID NO 7) or heterozygous (includes both SEQ ID NOs 7 and 8), while in cassette 2, QTL 4 is homozygous for allele "G" (SEQ ID NO 8) or heterozygous (includes both SEQ ID NOs 7 and 8).
SEQ ID NO 7 (SNP base bold): 
GGTGTATTTTACAATTTTATGCTGCTTTATGCTTAAGTGTCGTGGAACTCGCAAAGT
TTGGCTCCGCCACGAAATTTTGTACTATCGAGATTGTTATAAG A TATTGCTGAGAA
 
AGATCAAATCATGAGAAGACTGAACAAAAAGATTTTATTTCTGGATCAAATTCTGG
AGTGGAGAAGAGGAAAGCAGTGAGTGTGAAAA
SEQ ID NO 8 (SNP base bold):

GGTGTATTTTACAATTTTATGCTGCTTTATGCTTAAGTGTCGTGGAACTCGCAAAGT
TTGGCTCCGCCACGAAATTTTGTACTATCGAGATTGTTATAAG G TATTGCTGAGAA
AGATCAAATCATGAGAAGACTGAACAAAAAGATTTTATTTCTGGATCAAATTCTGG
AGTGGAGAAGAGGAAAGCAGTGAGTGTGAAAA

QTL 5, as used herein, refers to a polymorphic genetic locus linked to a genetic marker at position 8636698 on sesame chromosome 4. The two alleles of the genetic marker at QTL 5 have the SNP bases "C" or "T", as set forth respectively in the nucleic acid sequences of SEQ ID NOs 9 and 10. In cassette 3, QTL 5 is homozygous for allele "C" (SEQ ID NO 9) or heterozygous (includes both SEQ ID NOs 9 and 10), while in cassette 4, QTL 5 is homozygous for allele "T" (SEQ ID NO 10) or heterozygous (includes both SEQ ID NOs 9 and 10).
SEQ ID NO 9 (SNP base bold): 
CTTTTGTTATTTTACTTATTAAAAATTTAAAAAATAAATAAGAAGTGTAATTATTAT
TGTTTCATTTTTACCAAAAAGTTCGGTTTACCGAATACCGATT C GAAATTCCTAAA
CAGATTAGGTAGTATCTGTTCGGTATTCTGGTTCATTTGGATTTTACCTAACATCTGT
AACAACTCTTTTGGCCTTGGTGGTTCCAAT
SEQ ID NO 10 (SNP base bold):

CTTTTGTTATTTTACTTATTAAAAATTTAAAAAATAAATAAGAAGTGTAATTATTAT
TGTTTCATTTTTACCAAAAAGTTCGGTTTACCGAATACCGATT T GAAATTCCTAAA
CAGATTAGGTAGTATCTGTTCGGTATTCTGGTTCATTTGGATTTTACCTAACATCTGT
AACAACTCTTTTGGCCTTGGTGGTTCCAAT

QTL 6, as used herein, refers to a polymorphic genetic locus linked to a genetic marker at position 10189573 on sesame chromosome 4. The two alleles of the genetic marker at QTL 6 have the SNP bases "A" or "G", as set forth respectively in the nucleic acid sequences of SEQ ID NOs 11 and 12. In cassette 4, QTL 6 is homozygous for allele "A" (SEQ ID NO 11) or heterozygous (includes both SEQ ID NOs 11 and 12).
SEQ ID NO 11 (SNP base bold): 
AAAAACAAGATCATGCTTAATTCTATGGTCGATTATGTATGCAAAGACGACAATCA
TTCATGATAGATAAATTGGTTAT A ACCATTAGTACGACCTAACAACCTTACCTTTG
GACAGTCAAGGAACGTCGATTAACTAAATCCCTAATCAGTAAATAATC
SEQ ID NO 12 (SNP base bold):

AAAAACAAGATCATGCTTAATTCTATGGTCGATTATGTATGCAAAGACGACAATCA
TTCATGATAGATAAATTGGTTAT G ACCATTAGTACGACCTAACAACCTTACCTTTG
GACAGTCAAGGAACGTCGATTAACTAAATCCCTAATCAGTAAATAATC

QTL 7, as used herein, refers to a polymorphic genetic locus linked to a genetic marker at position 20018096 on sesame chromosome 1. The two alleles of the genetic marker at QTL 7 have the SNP bases "T" or "C", as set forth respectively in the nucleic acid sequences of SEQ ID NOs 13 and 14. In cassette 2, QTL 7 is homozygous for allele "T" (SEQ ID NO 13) or heterozygous (includes both SEQ ID NOs 13 and 14).
SEQ ID NO 13 (SNP base bold): 
GCGTAAAAGCATGACTTACGTTAAAAGCTACTACTAAAGTAAAATCCCCAATAGAG
TATTCTGTCTCATCTCAACCCATCGACTTTCCCATCCTCAACAT T TCCATCATTCTT
TCCGCCCTTTAAGCTATGGAAGGAACTAACTACTCCTGCTTAATTCCTAACCTCATC
TCAAAACACACTACCTGCGACTTGTAATGTT
SEQ ID NO 14 (SNP base bold):

GCGTAAAAGCATGACTTACGTTAAAAGCTACTACTAAAGTAAAATCCCCAATAGAG
TATTCTGTCTCATCTCAACCCATCGACTTTCCCATCCTCAACAT C TCCATCATTCTT
TCCGCCCTTTAAGCTATGGAAGGAACTAACTACTCCTGCTTAATTCCTAACCTCATC
TCAAAACACACTACCTGCGACTTGTAATGTT

QTL 8, as used herein, refers to a polymorphic genetic locus linked to a genetic marker at position 8384304 on sesame chromosome 4. The two alleles of the genetic marker at QTL 8 have the SNP bases "T" or "C", as set forth respectively in the nucleic acid sequences of SEQ ID NOs 15 and 16. In cassette 5, QTL 8 is homozygous for allele "T" (SEQ ID NO 15) or heterozygous (includes both SEQ ID NOs 15 and 16).
SEQ ID NO 15 (SNP base bold): 
TTTTGTTTGATAGGAGGAAAAGATGGATTCAAGTTTAGGCTGTGCATCACGCTGTAT
TCCCTATTAGATCACTGGCTGACCATGCAAGGACATCCATGTT C TCATGCAGGAAG
 
GTAGTGAATTCTCCTATTATTTCAAGGAATAGTTGGGAGCCGATCCGCCTCTCTTGA
TACAAGCTGGAGGCACCGTACCTCTTGAATG
SEQ ID NO 16 (SNP base bold):

TTTTGTTTGATAGGAGGAAAAGATGGATTCAAGTTTAGGCTGTGCATCACGCTGTAT
TCCCTATTAGATCACTGGCTGACCATGCAAGGACATCCATGTT T TCATGCAGGAAG
GTAGTGAATTCTCCTATTATTTCAAGGAATAGTTGGGAGCCGATCCGCCTCTCTTGA
TACAAGCTGGAGGCACCGTACCTCTTGAATG

QTL 9, as used herein, refers to a polymorphic genetic locus linked to a genetic marker at position 16918017 on sesame chromosome 8. The two alleles of the genetic marker at QTL 9 have the SNP bases "G" or "A", as set forth respectively in the nucleic acid sequences of SEQ ID NOs 17 and 18. In cassette 5, QTL 9 is homozygous for allele "G" (SEQ ID NO 17) or heterozygous (includes both SEQ ID NOs 17 and 18).
SEQ ID NO 17 (SNP base bold): 
CCGAAAGTAGTGAAGTCCAGCCTACTTTCGGTCCCGGTGGGCTCATAAGTCGATAA
ATATCGTGGGTCGTGGGCTTCCCACTAGTACACCTCCATACAAT G CGGTCGTCACC
CCCGTAAATCCTTTGCAAGGTATGTATAATCTCCATGTACTCCATATCCGTAATCAA
TGGCCAATGCCATTGTCCCTCATGAATAATGG
SEQ ID NO 18 (SNP base bold):

CCGAAAGTAGTGAAGTCCAGCCTACTTTCGGTCCCGGTGGGCTCATAAGTCGATAA
ATATCGTGGGTCGTGGGCTTCCCACTAGTACACCTCCATACAAT A CGGTCGTCACC
CCCGTAAATCCTTTGCAAGGTATGTATAATCTCCATGTACTCCATATCCGTAATCAA
TGGCCAATGCCATTGTCCCTCATGAATAATGG

QTL 10, as used herein, refers to a polymorphic genetic locus linked to a genetic marker at position 304411 on sesame chromosome 11. The two alleles of the genetic marker at QTL 10 have the SNP bases "T" or "C", as set forth respectively in the nucleic acid sequences of SEQ ID NOs 19 and 20. In cassette 6, QTL 10 is homozygous for allele "T" (SEQ ID NO 19) or heterozygous (includes both SEQ ID NOs 19 and 20).
SEQ ID NO 19 (SNP base bold): 
AGAAAAATAAGTAAAGGTATATGCTCGAATGCAGCAACGAGAGAAATCCTGGTTT
GCATG T TCTCCTAAATCAAGGCTGCAGTATATGGCGCCCACGCCAAGACTCCCTTC
CCTGAGCGAAC
SEQ ID NO 20 (SNP base bold):

AGAAAAATAAGTAAAGGTATATGCTCGAATGCAGCAACGAGAGAAATCCTGGTTT
GCATG C TCTCCTAAATCAAGGCTGCAGTATATGGCGCCCACGCCAAGACTCCCTTC
CCTGAGCGAAC

QTL 11, as used herein, refers to a polymorphic genetic locus linked to a genetic marker at position 11699161 on sesame chromosome 6. The two alleles of the genetic marker at QTL 11 have the SNP bases "T" or "C", as set forth respectively in the nucleic acid sequences of SEQ ID NOs 21 and 22. In cassette 7, QTL 11 is homozygous for allele "T" (SEQ ID NO 21) or heterozygous (includes both SEQ ID NOs 21 and 22).
SEQ ID NO 21 (SNP base bold): 
GGAGTGGTGCTCACCAGCCCAAAAGGGAACGAACTGGAATATGCTTTGCGTTTCGA
CTTTAGCGCTTCAAATAATGAGG C TGAATATTAAGCGCTTATTGGAGGTACCAGG
ATGGCCTTGAATACAAGAGCTAAAAGTCTAATTCCTACTCCGATTCCCA
SEQ ID NO 22 (SNP base bold):

GGAGTGGTGCTCACCAGCCCAAAAGGGAACGAACTGGAATATGCTTTGCGTTTCGA
CTTTAGCGCTTCAAATAATGAGG T TGAATATTAAGCGCTTATTGGAGGTACCAGG
ATGGCCTTGAATACAAGAGCTAAAAGTCTAATTCCTACTCCGATTCCCA

QTL 12, as used herein, refers to a polymorphic genetic locus linked to a genetic marker at position 13967471 on sesame chromosome 3. The two alleles of the genetic marker at QTL 11 have the SNP bases "C" or "T", as set forth respectively in the nucleic acid sequences of SEQ ID NOs 23 and 24. In cassette 7, QTL 12 is homozygous for allele "C" (SEQ ID NO 23) or heterozygous (includes both SEQ ID NOs 23 and 24).
SEQ ID NO 23 (SNP base bold): 
TCTAAAAGACACCCCAACAACTCGCTATTAAACAAATCAAGTATGATATGTGAGAG
TAGTGCTCTAATTATACCTACCTACTTTACAAACAAATTTGAAT T CCCTTGAAAGA
AATGATTGCCCTTTTATGCCTTTTGTTTTGTAATGCCTCAGGTTTAATATTAATAATA
CAGACACTCACCCCCAAGATTCATTTCTCTA
SEQ ID NO 24 (SNP base bold):

TCTAAAAGACACCCCAACAACTCGCTATTAAACAAATCAAGTATGATATGTGAGAG
TAGTGCTCTAATTATACCTACCTACTTTACAAACAAATTTGAAT C CCCTTGAAAGA

AATGATTGCCCTTTTATGCCTTTTGTTTTGTAATGCCTCAGGTTTAATATTAATAATA
CAGACACTCACCCCCAAGATTCATTTCTCTA

Sesame plant with shatter-resistant capsules, progeny thereof and/or part(s) thereof are provided, which comprise a plurality of quantitative trait loci (QTLs) having a corresponding plurality of nucleic acid genetic markers that are associated with a plurality of phenotypic traits of the sesame plant comprising at least one of: a taste trait, a protein content trait and an amino acid composition trait, wherein the QTLs are combined in the sesame plant from a plurality of sesame varieties by computationally supported breeding.

The QTLs of disclosed sesame plants, progeny thereof and/or part(s) thereof, may comprise QTL 1 associated with improved taste, with corresponding markers set forth in SEQ ID NOs 1 or 2, wherein the sesame plant or part thereof is homozygous or heterozygous with respect to SEQ ID NO 1.

Disclosed sesame plants with shatter-resistant capsules (and/or progeny thereof, seeds and/or part(s) thereof) may comprise, in addition to QTL 1, at least one additional QTL, and the markers may be arranged in cassettes comprising at least one of: (i) cassettes 1, 2, 3 or 4, comprising at least QTL 1, and QTL 2 associated with lowered ash content and increased protein level, with corresponding markers set forth in SEQ ID NOs 3 or 4, and wherein the sesame plant or part thereof is homozygous or heterozygous with respect to SEQ ID NO 3; (ii) cassette 5 comprising at least QTL 1, and QTLs 8 and 9 associated with high protein content, with QTL 8 having corresponding markers set forth in SEQ ID NOs 15 or 16, wherein the sesame plant or part thereof is homozygous or heterozygous with respect to SEQ ID NO 15, and with QTL 9 having corresponding markers set forth in SEQ ID NOs 17 or 18, wherein the sesame plant or part thereof is homozygous or heterozygous with respect to SEQ ID NO 17; cassette 6 comprising at least QTL 1, and QTL 10 associated with improved taste, with corresponding markers set forth in SEQ ID NOs 19 or 20, wherein the sesame plant or part thereof is homozygous or heterozygous with respect to SEQ ID NO 19; and/or cassette 7 comprising at least QTL 1, and QTL 11 and QTL 12 associated with high protein content, with QTL 11 having corresponding markers set forth in SEQ ID NOs 21 or 22, wherein the sesame plant or part thereof is homozygous or heterozygous with respect to SEQ ID NO 21 and with QTL 12 having corresponding markers set forth in SEQ ID NOs 23 or 24, wherein the sesame plant or part thereof is homozygous or heterozygous with respect to SEQ ID NO 23.

The QTLs of disclosed sesame plants, progeny thereof and/or part(s) thereof, may comprise QTL 2 associated with high protein content, above 29.8% dry matter weight percent, as well as low content of ash, with corresponding markers set forth in SEQ ID NOs 3 or 4, wherein the sesame plant or part thereof is homozygous or heterozygous with respect to SEQ ID NO 3.

The QTLs of disclosed sesame plants, progeny thereof and/or part(s) thereof, may comprise QTL 3 associated with high methionine content, with corresponding markers set forth in SEQ ID NOs 5 or 6, wherein the sesame plant or part thereof is homozygous or heterozygous with respect to SEQ ID NO 5.

The QTLs of disclosed sesame plants, progeny thereof and/or part(s) thereof, may comprise QTL 4 associated with high methionine content, with corresponding markers set forth in SEQ ID NOs 7 or 8, wherein the sesame plant or part thereof is homozygous or heterozygous with respect to SEQ ID NO 7.

In some embodiments, disclosed sesame plants, progeny thereof and/or part(s) thereof may comprise disclosed marker cassette 1 which comprises QTLs 1, 2, 3 and 4 as disclosed herein.

The QTLs of disclosed sesame plants, progeny thereof and/or part(s) thereof, may comprise QTL 7 associated with high protein content, with corresponding markers set forth in SEQ ID NOs 13 or 14, and wherein the sesame plant or part thereof is homozygous or heterozygous with respect to SEQ ID NO 13.

In some embodiments, disclosed sesame plants, progeny thereof and/or part(s) thereof may comprise disclosed marker cassette 2 which comprises QTLs 1, 2, 3, 4 and 7 as disclosed herein.

The QTLs of disclosed sesame plants, progeny thereof and/or part(s) thereof, may comprise QTL 5 associated with high methionine content, with corresponding markers set forth in SEQ ID NOs 9 or 10, and wherein the sesame plant or part thereof is homozygous or heterozygous with respect to SEQ ID NO 9.

In some embodiments, disclosed sesame plants, progeny thereof and/or part(s) thereof may comprise disclosed marker cassette 3 which comprises QTLs 1, 2, 3 and 5 as disclosed herein.

The QTLs of disclosed sesame plants, progeny thereof and/or part(s) thereof, may comprise QTL 6 associated with high protein content, with corresponding markers set forth in SEQ ID NOs 11 or 12, wherein the sesame plant or part thereof is homozygous or heterozygous with respect to SEQ ID NO 11.

In some embodiments, disclosed sesame plants, progeny thereof and/or part(s) thereof may comprise disclosed marker cassette 4 which comprises QTLs 1, 2, 3, 5 and 6 as disclosed herein.

The QTLs of disclosed sesame plants, progeny thereof and/or part(s) thereof, may comprise QTL 8 associated with high protein content, with corresponding markers set forth in SEQ ID NOs 15 or 16, wherein the sesame plant or part thereof is homozygous or heterozygous with respect to SEQ ID NO 15.

The QTLs of disclosed sesame plants, progeny thereof and/or part(s) thereof, may comprise QTL 9 associated with high protein content, with corresponding markers set forth in SEQ ID NOs 17 or 18, and wherein the sesame plant or part thereof is homozygous or heterozygous with respect to SEQ ID NO 17.

In some embodiments, disclosed sesame plants, progeny thereof and/or part(s) thereof may comprise disclosed marker cassette 5 which comprises QTLs 1, 8 and 9 as disclosed herein

The QTLs of disclosed sesame plants, progeny thereof and/or part(s) thereof, may further comprise QTL 10 associated with improved taste, with corresponding markers set forth in SEQ ID NOs 19 or 20, wherein the sesame plant or part thereof is homozygous or heterozygous with respect to SEQ ID NO 19.

In some embodiments, disclosed sesame plants, progeny thereof and/or part(s) thereof may comprise disclosed marker cassette 6 which comprises QTLs 1 and 10 as disclosed herein.

In some embodiments, disclosed sesame plants, progeny thereof and/or part(s) thereof may comprise disclosed marker cassette 7 which comprises QTLs 1, 11 and 12 as disclosed herein.

In various embodiments, disclosed sesame plants, progeny thereof and/or part(s) thereof, may comprise any of the QTLs disclosed herein, or combinations thereof. In various embodiments, disclosed sesame plants, progeny thereof and/or part(s) thereof, may comprise any of the QTL cassettes disclosed herein, or combinations thereof.

**Figure 2A** is a high-level schematic illustration of a computationally supported breeding method **200,** according to some embodiments of the invention. Method **200** may be at least partially implemented by at least one computer processor; computationally supported breeding method **200** is used to detect and combine QTLs from a plurality of sesame varieties to develop disclosed high protein sesame plants with shatter-resistant capsules which are different than any of the parent varieties by virtue of the achieved phenotypical characteristics.

Computationally supported breeding method **200** comprises stages of trait discovery by growing and phenotyping a broad spectrum of varieties (stage **210),** trait blending by developing hybridized lines through crossing the selected lines to mix and combine traits and selfing of the progeny in subsequent generations (stage **220),** Target Product Genomic Code (TPGC) discovery by associating phenotypes and genotypes using derived linkage maps (stage **230),** *in silica* selection to suggest candidate varieties (stage **240),** breeding candidate varieties and selection of varieties based on the best TPGC potential (stage **250)** and genomic code (GC) discovery to identify the most stable QTLs in hybridized progeny generation(s) (stage **260),** as explained in detail below. TPGC discovery **230,** *in silica* validation **250** and GC discovery **260** are based on computational algorithms that cannot be performed manually and provide the computational support for the judicious selection of the varieties that are generated and further crossed during the development process to yield disclosed high protein sesame plants with shatter-resistant capsules. It is noted that during the discovery phase, the QTLs are derived in order to combine them (by hybridization) to create unique combinations of QTLs which do not exist in known world lines.

In certain embodiments, sesame lines were bred to reach high protein levels by collecting various sesame lines worldwide, creating F2 linkage populations, applying intensive phenotyping and genotyping of thousands of sesame lines, predicting of QTLs affecting the protein level and/or composition traits, and establishing unique marker combinations, termed "marker cassettes" herein, to characterize novel high protein lines provided by the methods described herein and not existing in commercial or natural lines.

The breeding methodology was based on algorithms for deriving the Target Product Genomic Code (TPGC) to associate (i) the Target Product (TP) being defined in advance based on market requirements and including a set of desired attributes (traits) that are available in natural genetic variations; and (ii) the Genomic Code (GC) comprising set(s) of genomic regions that include quantitative trait loci (QTLs) that affect and are linked to the TP traits. The algorithms may be configured to calculate multiple genomic interactions and to maximize the genomic potential of specific plants for the development of new varieties. The breeding program was constructed to derive the TPGC, and then by crossing and selfing to achieve a product which contains the specific GC that corresponds to the required TPs.

Certain embodiments of the breeding process of developing lines, through crossing and successive generations of selfing comprise stages such as: (i) Trait Discovery, in which a broad spectrum of varieties from different geographies and worldwide sources are grown and phenotyped in order to discover new traits that can potentially be combined to create new varieties; (ii) Trait Blend, in which a crossing cycle is carried out based on phenotypic assumption(s), in which the different traits are mixed and combined. Initial trait cycle(s) are followed by additional cycle(s) to create F2 (and possibly higher generations) population(s) that provide the basis for algorithmic analysis for constructing the TPGC; (iii) TPGC Discovery, in which the plant(s) are phenotyped and genotyped to produce linkage map(s), discovering the relevant QTLs and deriving the TPGC; (iv) several line validation stages over several years in which sesame lines based on millions of *in silica* calculated variations (and/or selections) are grown and are used to define the initial varieties; (v) Trait TPGC Blend, in which accurate crossings are performed in order to calculate the most efficient way to reach the best TPGC. The crossings are performed after *in silica* selection from millions of combinations, and are based, at least in part on phenotype assumptions; and (vi) Consecutive algorithm-based GC discovery stage(s) applied to F2 (or higher generation) population(s) grown in additional cycle(s).

Defining the TP for high protein shatter-resistant sesame varieties includes the development of high throughput methods for high protein level identification.

In the following non-limiting example of the process, Trait Discovery (i) was based on proprietary germplasm including hundreds of elite varieties and thousands of F2 individual plants and also ca. 200 different sesame lines that were obtained from the U.S. National Plant Germplasm System (NPGC) and courtesy of professor Amram Ashri's sesame germplasm collection (see Ashri, A. 1998, Sesame Breeding. In: Janick J. (ed.), Plant Breeding Reviews Vol. 16. John Wiley and Sons, Somerset, NJ, pp. 179-228). These lines were used for the Trait Blend stage (ii), with crosses executed based on the potential for enrichment of genomic diversity to create new complex(es) of traits for the high protein level as the initial step for the TP-directed breeding program for high protein sesame lines. The resulting F1 hybrids were later self-crossed to create F2 linkage populations that showed phenotypic segregation. The F2 populations were then planted in three different environments for discovering the TPGC (iii) that includes high protein, amino acids composition and taste traits. After screening and deep phenotyping of 2500 individuals, a set of ca. 300 representatives was selected. The selected individuals from the F2 populations were further massively phenotyped for traits associated with protein level, amino acids fractional composition and taste, as detailed in the following description. The measurement results were summarized into the representative high protein traits.

TPGC Discovery (iii) included genotyping ca. 2500 selected individual plants from six populations. The analysis was performed with a panel of 1000 markers based on single nucleotide polymorphism (SNP) and directly designed based on the polymorphism found in the parental lines of the populations which were analyzed in depth using high throughput DNA sequencing technologies. The panel was designed to maximize the chance of having the largest number of common segregating SNPs in order to create highly similar linkage maps for all observed populations (e.g., "consensus map"). The computation of linkage maps was executed on each linkage F2 population based on the genotyping results. Linkage maps were computed with MultiPoint, an interactive package for ordering multi loci genetic maps, and verification of maps was based on resampling techniques. Discovery of QTLs that are related to high protein level was carried out with the MultiQTL package, based on the linkage maps that were merged by Multipoint and the F2 population phenotype data, and using multiple interval mapping (MIM). The significance and co-occurrences of the high protein level and protein content markers were evaluated using an algorithm that related the genotype-phase of each marker to respective QTLs and traits in linkage maps of the six F2 populations (also called "linkage F2 populations" herein) in each population, for populations in different environments. QTL significance was computed with permutation, bootstrap tools and FDR (false discovery rate) for total analysis. The linkage maps of all six F2 populations and the information of the high protein, amino acid composition and taste traits over all genotyped plants belonging to those populations were analyzed and used to predict the QTLs in a "one trait to one marker" model, in which for all markers that constructed the linkage maps, each trait was tested independently against each one of the markers. Specifically, a subset of two populations (ca. 800 F2 individuals) was tasted for the discovery of taste QTLs, which were then validated within a smaller subset of advanced lines and applying tasting test at a higher resolution (using more tasters for fewer lines). In the provided examples, altogether ten markers were found to be related to traits associated with protein-related components (see **Table 1** above), with one taste marker (QTL 1) common to all cassettes and another high protein marker (QTL 2) common to four of the cassettes. The occurrence of high protein level markers in two or more linkage maps of the F2 population (repetitive markers) strengthened its significance as representative for high protein level QTL.

In general, the six linkage F2 populations presented different markers that related to high protein levels. However, subsets of common markers were found to be shared by multiple populations and are referred to herein as marker cassettes.

It is emphasized that the breeding process is explained using non-limiting examples from a specific part of the breeding program, and is not limited to the specific populations and varieties derived by this specific part of the breeding program. For example, different F2 population may be bred and used to derive additional varieties that are characterized by one or more of the disclosed QTLs.

Following TPGC Discovery (iii), an *in-silico* breeding program (iv) was established to process the TPGC blend (including combinations of QTLs for different plants) to simulate and predict the genotypic states of self, cross-self and hybrid plant with respect to the QTLs and their predicted effects on each phase of the markers for the high protein level trait. The *in-silico* breeding program was constructed to yield millions of *in silica* selfing combinations which were bred and evaluated *in-silico* up to F8 - to measure the potential for each of the genotyped plants to acquire the high protein level in the right combination at the right phase. The analysis resulted in identifying ca. 300 F2 plants having the highest score for high protein level, which were thus chosen for the actual selfing and cross-selfing procedures. The F3 seeds from these selected F2 plants were sown in plots in the subsequent growing season. Under this procedure, QTLs from different populations were combined to yield F3 plants containing new and unique cassettes of QTLs and resulting in high protein levels.

The high protein sesame lines were then validated as retaining the trait in the following generations by genotyping the F3 and some subsequent generation offspring to verify they maintained the identified marker cassettes. Specifically, the parental lines of linkage F2 populations together with ca. 200 different sesame cultivars (landraces and old commercial varieties) were genotyped based on high protein level markers of all populations. The cassettes detailed in **Table 1** were found to wholly differentiate the developed high protein lines from the rest of the sesame cultivars screened.

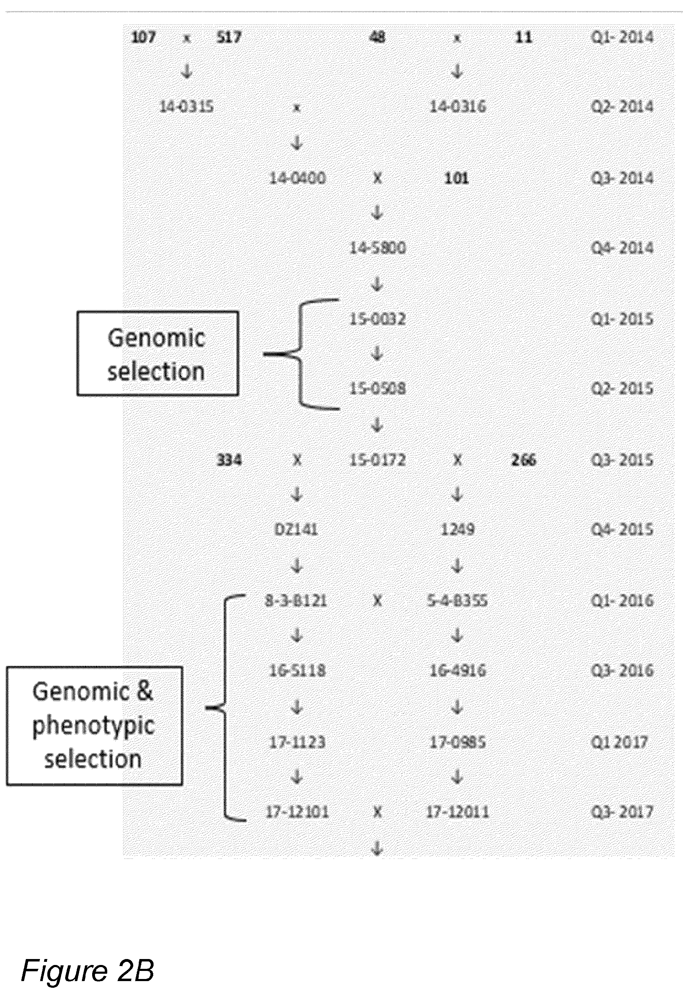

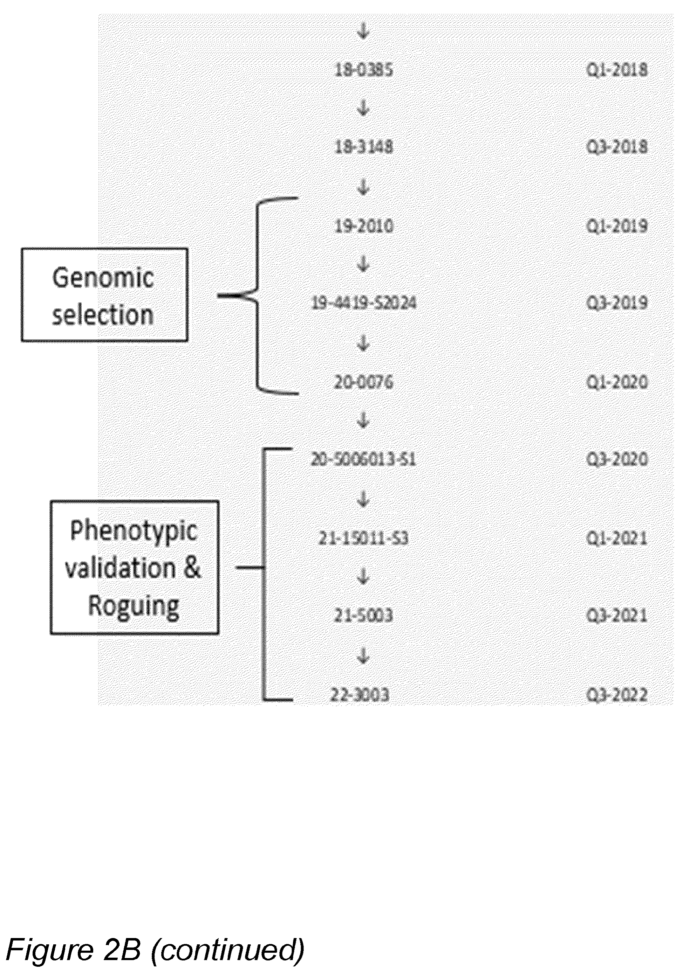

**Figure 2B** provides a non-limiting example of the breeding process and genealogy of one of the disclosed varieties, developed using the disclosed methodology (see annotated stages), according to some embodiments of the invention. Specifically, **Figure 2B** illustrates the initial progeny lines (denoted 107, 517, 48 and 11 by their accession serial numbers, with corresponding USDA Identifiers PI 599447, PI 173955, PI 254699 and PI 157158) and the consecutive progeny lines (denoted 101, 334, 266 by their accession serial numbers, with corresponding USDA Identifiers PI 599438, PI 532845 and PI 263470) which were involved in the breeding of one of the varieties (denoted ES309) that were characterized above as including marker cassette 7. It is noted that the breeding process was carried out according to method 200, involving an extensive process of trait discovery and blending via *in silica* selection among a very large number of possible combinations, as well as the actual extended breeding over eight years, including judicial crossings that were based on the computerized analysis and stabilization of selected varieties.

The inventors note that none of the high protein sesame plants with shatter-resistant capsules that were bred according to the methods described herein is naturally occurring; indeed, they were derived by highly complicated computationally-supported breeding methods **200** described above, in which the genotypes of multiple sesame varieties were judiciously combined and analyzed, to discover and accumulate the recited QTL markers and corresponding phenotypic traits. Although the recited sesame plants are not genetically modified by sequences originating from other species, they cannot be reached merely by natural processes, as is evident by the detailed and intentional breeding program that was applied to specifically measure required characteristics, detect corresponding markers using bioinformatics methods and combine the detected QTLs in the selected varieties by classic breeding approaches. The inventors note that due to the huge complexity of the breeding program, involving growing, selecting and breeding of hundreds of varieties over many generations in the field, and based on genetic analysis of the varieties and of the relations of markers to phenotypic characteristics, this breeding process cannot happen merely by natural means and therefore cannot be considered a natural phenomenon. It is further noted that due to the shatter-resistance characteristics, disclosed varieties are severely hindered from natural plant propagation. Finally, it is noted that while the disclosed QTL markers are not heterologous to sesame as a species, the identified QTLs are not present in the recited combinations in any of over 200 prior art varieties which were used as initial breeding stock, and that the QTLs genomics of the sesame plants has been significantly and judiciously modified by the breeding program. Therefore, at the taxonomic level of the varieties, the high protein sesame plants may be considered hybridized in that the QTL markers are mixed and introduced from other sesame varieties.

In most of the food products, the inventors have found out that the disclosed markers are still present and can be detected in the food products, as indicated by the following experimental results, pertaining to prior art markers. The data illustrates that various markers, relating to various sesame varieties, some of which disclosed herein and some of which disclosed in previous patents by the Applicant as specified below - are retained in processed food products and provides a robust illustration of the traceability of the disclosed markers in the disclosed food products made of disclosed sesame seeds. Specifically, the experimental data indicates that as genetical markers are generally maintained in food products such as de-hulled seeds and tahini prepared from sesame seeds, the disclosed markers are also maintained or expected to be maintained in food products prepared from the disclosed varieties. The following results therefore illustrate that genetic markers are retained in food products prepared from sesame seeds, even under harsh conditions such as heating and milling, irrespective of the exact marker sequence, which is understood to be irrelevant with respect to the biochemical and physical processes that modify the seeds during their processing. Similar processing stages are applied to many types of sesame-based food products, and hence the provided results also indicate that the disclosed markers are likewise retained in the variety of disclosed food products made with the disclosed seeds, including - dehulled (non-viable and non-germinating) sesame seeds, packaged and sold as seeds, used as topping and/or added to a variety of baked goods or fried goods (e.g., buns, bagels, snacks, etc.), food and protein bars, granola mixtures and granola bars, various types of candy, various types of spreads (e.g., tahini, hummus, etc.), confectionary foods (e.g., halva), various food products prepared from ground or broken sesame, sesame flour and/or sesame oil, as well as sesame foul and sesame oil themselves. Following initial investigation, all processed sesame retains detectable genetic markers disclosed herein, probably except for products which are processed under even greater heat than tested herein (e.g., sesame oil), as heat is expected to degrade genetic material.

The following results illustrate the ability to detect the disclosed genetic markers in processed sesame products and downstream commercial products. The evaluation was carried out, in a non-limiting manner, using various seeds varieties by the Applicant, and were compared with a control prior art Humera variety. Specifically, the genetical analysis was carried out for de-hulled seeds on one sesame variety including a cassette of markers disclosed in U.S. Patent Application Publication No. 2021/0400901; two sesame varieties including respective two cassettes of markers disclosed in U.S. Patents Nos. 10,577623 and 11,445,692; two sesame varieties including respective two cassettes of markers disclosed in U.S. Patents Nos. 11,044,884 and 11,730,133; one sesame variety including a cassette of markers disclosed in U.S. Patent No. 11,395,470; and two sesame varieties including respective two cassettes of markers disclosed herein in the current Application. The genetical analysis was carried out for tahini on one sesame variety including a cassette of markers disclosed in U.S. Patent Application Publication No. 2021/0400901; three sesame varieties including respective two cassettes of markers disclosed in U.S. Patents Nos. 10,577623 and 11,445,692; one sesame variety including a cassette of markers disclosed in U.S. Patents Nos. 11,044,884 and 11,730,133; one sesame variety including a cassette of markers disclosed in U.S. Patent No. 11,395,470; and one sesame variety including a cassette of markers disclosed herein in the current Application.

The respective seeds of the tested sesame varieties were processed into de-hulled sesame and into tahini, which are two representative commercial forms. For de-hulled sesame seeds, respective varieties were de-hulled under laboratory conditions - by soaking seeds in water, releasing the hulls by mixing, separating husks, and rinsing and drying until completely free of hulls. For tahini, respective varieties were processed under industrial conditions - sesame seeds were de-hulled using a wet de-hulling process, roasted at 110 °C for approximately 1 hour, and ground to a fine paste using a modern industrial grinding system. The tahini was produced on a commercial production line typical of large-scale manufacturers.

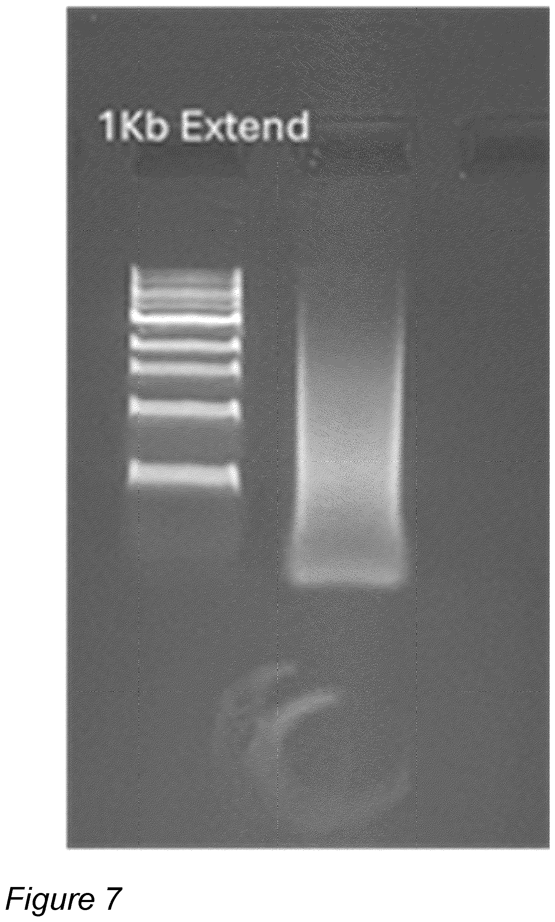

Both product types were subjected to marker screening assays for the respective cassettes of markers using Quick-DNA Plant/Seed Miniprep Kit with a relevant amplicon. **Figure 7** illustrates the retention and detection of genomic DNA in tahini following industrial processing, according to some embodiments of the invention. Specifically, **Figure 7** illustrates the detection of genomic DNA on 1% agarose gel after DNA was extracted from tahini made of disclosed sesame seeds under industrial conditions. The left-hand strap illustrates a DNA ladder indicating the proper operation of the analysis procedure, and the right-hand straps illustrates a DNA smear that indicates that a broad range of DNA fragment sizes have been successfully extracted from the tahini. These fragments were then amplified by amplicon sequencing and analyzed to check whether the specific markers in each cassette are detected in the food product.

In all varieties listed above for either product, the respective markers in each respective cassette were positively detected in the food product prepared from the respective seeds, while the control sample prepared from prior art Humera variety did not include any of the respective markers. It is concluded that disclosed food products made of disclosed seeds have at least one of the disclosed markers that is detectable in the food product.

Together, the results from de-hulled sesame and tahini demonstrate that the disclosed genetic markers are stable and detectable throughout multiple industrial processing steps. These results also establish the persistence of disclosed markers in processed food products made of sesame, which allow authentication and traceability of both raw and processed sesame products with respect to the seeds from which they were prepared.

In the above description, an embodiment is an example or implementation of the invention. The various appearances of "one embodiment", "an embodiment", "certain embodiments" or "some embodiments" do not necessarily all refer to the same embodiments. Although various features of the invention may be described in the context of a single embodiment, the features may also be provided separately or in any suitable combination. Conversely, although the invention may be described herein in the context of separate embodiments for clarity, the invention may also be implemented in a single embodiment. Certain embodiments of the invention may include features from different embodiments disclosed above, and certain embodiments may incorporate elements from other embodiments disclosed above. The disclosure of elements of the invention in the context of a specific embodiment is not to be taken as limiting their use in the specific embodiment alone. Furthermore, it is to be understood that the invention can be carried out or practiced in various ways and that the invention can be implemented in certain embodiments other than the ones outlined in the description above.

The invention is not limited to those diagrams or to the corresponding descriptions. For example, flow need not move through each illustrated box or state, or in exactly the same order as illustrated and described. Meanings of technical and scientific terms used herein are to be commonly understood as by one of ordinary skill in the art to which the invention belongs, unless otherwise defined. While the invention has been described with respect to a limited number of embodiments, these should not be construed as limitations on the scope of the invention, but rather as exemplifications of some embodiments. Other possible variations, modifications, and applications are also within the scope of the invention. Accordingly, the scope of the invention should not be limited by what has thus far been described, but by the appended claims and their legal equivalents.

## Claims

1. A food product prepared with seeds of a sesame plant with shatter-resistant capsules, progeny thereof, seeds and/or part(s) thereof, the sesame plant comprising:
a plurality of quantitative trait loci (QTLs) having a corresponding plurality of nucleic acid genetic markers that are associated with a plurality of phenotypic traits concerning taste and nutritional content of the seeds, wherein the QTLs are combined in the sesame plant from a plurality of sesame varieties by computationally supported breeding,
wherein the QTLs comprise QTL 1 associated with improved taste, with corresponding markers set forth in SEQ ID NOs 1 or 2, wherein the sesame plant or part thereof is homozygous or heterozygous with respect to SEQ ID NO 1 and at least one additional QTL, and
wherein the markers are arranged in cassettes comprising at least one of:
cassettes 1, 2, 3 or 4, comprising at least QTL 1, and QTL 2 associated with lowered ash content and increased protein level, with corresponding markers set forth in SEQ ID NOs 3 or 4, and wherein the sesame plant or part thereof is homozygous or heterozygous with respect to SEQ ID NO 3;
cassette 5 comprising at least QTL 1, and QTLs 8 and 9 associated with high protein content, with QTL 8 having corresponding markers set forth in SEQ ID NOs 15 or 16, wherein the sesame plant or part thereof is homozygous or heterozygous with respect to SEQ ID NO 15, and with QTL 9 having corresponding markers set forth in SEQ ID NOs 17 or 18, wherein the sesame plant or part thereof is homozygous or heterozygous with respect to SEQ ID NO 17;
cassette 6 comprising at least QTL 1, and QTL 10 associated with improved taste, with corresponding markers set forth in SEQ ID NOs 19 or 20, wherein the sesame plant or part thereof is homozygous or heterozygous with respect to SEQ ID NO 19; and
cassette 7 comprising at least QTL 1, and QTL 11 and QTL 12 associated with high protein content, with QTL 11 having corresponding markers set forth in SEQ ID NOs 21 or 22, wherein the sesame plant or part thereof is homozygous or heterozygous with respect to SEQ ID NO 21 and with QTL 12 having corresponding markers set forth in SEQ ID NOs 23 or 24, wherein the sesame plant or part thereof is homozygous or heterozygous with respect to SEQ ID NO 23;
wherein the cassettes comprise at least one of cassettes 1, 2, 3 and/or 4, and
wherein at least one of the markers is detectable in the food product.

2. The food product of claim 1, wherein the cassettes further comprise at least one of cassette 5, 6 and/or 7

3. The food product of claim 1 or 2, wherein the QTLs further comprise QTL 3 associated with high methionine content, with corresponding markers set forth in SEQ ID NOs 5 or 6, and wherein the sesame plant or part thereof is homozygous or heterozygous with respect to SEQ ID NO 5.

4. The food product of any of claims 1-3, comprising cassette 1 with the QTLs further comprising QTL 4 associated with high methionine content, with corresponding markers set forth in SEQ ID NOs 7 or 8, and wherein the sesame plant or part thereof is homozygous or heterozygous with respect to SEQ ID NO 7.

5. The food product of any of claims 1-4, comprising cassette 2 with the QTLs further comprising QTLs 4 and 7 associated with high methionine content, wherein QTL 4 has corresponding markers set forth in SEQ ID NOs 7 or 8, and the sesame plant or part thereof is homozygous or heterozygous with respect to SEQ ID NO 8, and QTL 7 has corresponding markers set forth in SEQ ID NOs 13 or 14, and wherein the sesame plant or part thereof is homozygous or heterozygous with respect to SEQ ID NO 13.

6. The food product of any of claims 1-5, wherein the QTLs further comprise QTL 3 associated with high methionine content, with corresponding markers set forth in SEQ ID NOs 5 or 6, and wherein the sesame plant or part thereof is homozygous or heterozygous with respect to SEQ ID NO 6.

7. The food product of claim 6, comprising cassette 3 with the QTLs further comprising QTL 5 associated with high methionine content, with corresponding markers set forth in SEQ ID NOs 9 or 10, and wherein the sesame plant or part thereof is homozygous or heterozygous with respect to SEQ ID NO 9.

8. The food product of any of claims 1-7, comprising cassette 4 with the QTLs further comprising QTL 5 associated with high methionine content, with corresponding markers set forth in SEQ ID NOs 9 or 10, and wherein the sesame plant or part thereof is homozygous or heterozygous with respect to SEQ ID NO 10, and QTL 6 associated with low carbohydrate content, with corresponding markers set forth in SEQ ID NOs 11 or 12, and wherein the sesame plant or part thereof is homozygous or heterozygous with respect to SEQ ID NO 11.

9. The food product of any of claims 1-8, wherein the seeds have a protein content higher by at least 5% and taste improved by at least one scale grade, compared to the Muganli-57 sesame variety, when grown under the same conditions.

10. The food product of claim 9, wherein the seeds have a taste improved by at least two scale grades, compared to the Muganli-57 sesame variety, when grown under the same conditions.

11. The food product of any of claims 1-10, wherein the food product comprises at least one of: non-viable sesame seeds, dehulled seeds, baked or fried goods including and/or topped with the sesame seeds or dehulled seeds, food and protein bars including the seeds, granola mixes and food bars including the seeds, tahini and spreads, dips and sauces made of or including the seeds, alternative milk products made of or including the seeds, and halva, candy and confectionary made of or including the seeds.
